# EUROPEAN PATENT SPECIFICATION

(11) **EP 4 153 779 B1**
(45) Date of publication and mention of the grant of the patent: **25.02.2026**
(21) Application number: 21791708.7
(22) Date of filing: 26.04.2021
(51) Int. Cl.: C12Q 1/6869, G01N 21/64, C12N 9/14

(54) **FLUORESCENT POLYNUCLEOTIDE SEQUENCING METHODS AND COMPOSITIONS**
FLUORESZENZPOLYNUKLEOTID-SEQUENZIERUNGSVERFAHREN UND ZUSAMMENSETZUNGEN
PROCÉDÉS ET COMPOSITIONS DE SÉQUENÇAGE DE POLYNUCLÉOTIDES FLUORESCENTS

(30) Priority: 24.04.2020 US 202063015250 P
(43) Date of publication of application: 29.03.2023
(73) Proprietor: Quantapore, Inc., South San Francisco, CA 94080 (US)
(72) Inventor: HUBER, Martin, Menlo Park, California 94025 (US); ASSAD, Ossama, Redwood City, California 94063 (US); CLEEK, Terry, Menlo Park, California 94025 (US); DAVLIEVA, Milya, Alameda, California 94502 (US)
(74) Representative: TLIP Limited
(86) International application number: PCT/US2021/029210
(87) International publication number: WO 2021/217146

(56) References cited:
- WO-A1-2017/180319
- WO-A1-2018/195222
- CN-C- 100 445 397
- US-A- 4 962 037
- US-A1- 2019 002 971
- US-A1- 2019 002 971
- US-A1- 2020 096 493
- US-B2- 8 845 880

## Description

### CROSS-REFERENCE TO RELATED APPLICATIONS

This application claims the benefit of priority to co-owned U.S. Provisional Patent Application No. 63/015,250 filed on April 24, 2020.

### FIELD OF THE INVENTION

The inventions relate to nucleic acid sequencing.

### INTRODUCTION

The determination of the nucleotide sequences of nucleic acids, especially RNA and DNA, has been critical to advancing biological and medical research. Many methods have been proposed over the past several decades to facilitate nucleic acid sequencing. Two early methods, which were developed by Maxam and Gilbert (using base-selective chemical degradation procedures) and Sanger (using 3'-blocked nucleotide triphosphates referred to as "3'-terminators"), were widely used in research laboratories in the 1980's and 1990's. However, these methods were inconvenient due to their reliance on delicate polyacrylamide slab gels to resolve radioactively labeled nucleic acid fragments in adjacent lanes (4 lanes per sequence, one for each of the standard four nucleic acid bases types (A, C, G and T), and the need for lengthy film exposure times to generate images of sequencing fragments (also called sequencing ladders) from which sequences could be assembled.

In the 1990's, researchers adapted the Sanger method to a non-radioactive detection approach using sequencing fragments containing fluorescent labels that were generated using either fluorescently labeled primers (fluorescent primer method) or fluorescently labeled dideoxy nucleotide triphosphates (fluorescent terminator method). The fluorescent terminator method, using multiplex capillary electrophoresis to separate sequencing fragments, was the primary method used to determine the first human genome sequences published in 2001 by Celera and a consortium of academic laboratories.

Since 2001, several so-called "next generation" sequencing methods have been developed. The dominant sequencing method for the last ten years has been a sequencing-by-synthesis method developed by Solexa and commercialized by Illumina. In the Solexa method, sequencing data are collected while a complementary strand is synthesized by primer extension along a complementary sample template strand. In this method, a primer is hybridized to an immobilized template strand, forming a primer-template complex. The hybridized primer is extended by a DNA polymerase in the presence of four different fluorescently labeled reversibly 3' blocked nucleotide terminators (dNTPs). The polymerase catalyzes the addition of a nucleotide terminator that is complementary to the nucleotide base in the template, to the 3'-end of the growing primer, thereby creating a 3'-blocked primer. After residual blocked dNTPs are removed, the primer-template complex is imaged to determine which type of dNTP was added to the primer. After image collection, the fluorescent label of the added dNTP is removed, and the 3'-carbon of the added dNTP is de-blocked to generate a de-blocked primer having an extendable 3'-hydroxyl group. The de-blocked primer is then ready for further cycles of adding, imaging, and de-blocking/de-labeling to determine the sequence of the template strand from the sequence of complementary dNTPs that were added to the primer. Although this method has provided high throughput with good accuracy, it requires the alignment of large numbers of short sequences, leaving significant sequence gaps and sequence ambiguities. Moreover, it is expensive and requires extensive time-consuming sample preparation.

A different sequencing-by-synthesis method developed by Pacific Biosciences uses gamma labeled fluorescent dNTPs that are added by a template-dependent DNA polymerase to the 3' end of an extendable primer. As each dNTP is added, the gamma label is simultaneously cleaved from the NTP and detected. Since this method requires that the polymerase be held closely to the bottom of a reaction chamber illuminated by a high intensity evanescent field, the polymerase, template, and nearby labeled NTPs suffer photodamage that can inactivate the polymerase by photo-induced crosslinking and can destroy the gamma labels by photobleaching. Another limitation is that due to the need to preload each DNA polymerase molecule with a DNA template prior to the polymerase being immobilized on the bottom of each reaction chamber, each reaction chambers can only be used once for a single sequence read.

Oxford Nanopore Technologies has commercialized a sequencing technology that involves measuring transmembrane electrical currents while a single-stranded sample nucleic acid passes through a protein nanopore. The sequence of the nucleic acid is deduced from changes in the measured currents, which depend on the particular nucleotide sequences passing through the nanopore at a particular time. However, this method has not been widely adopted due to a number of factors. The method typically uses protein nanopores, which require hydrophobic environments, such as lipid bilayers, that are unstable and are difficult to manufacture. Translocation speeds that are too fast or inconsistent can cause nucleotides to be mis-identified or missed entirely. These issues are not fully solved when so-called molecular motors are included to slow translocation. Also, sequence determinations by this method usually require complicated algorithms to deconvolute the contributions of individual nucleotides to measured currents that have simultaneous contributions from multiple (e.g., five or more) consecutive nucleotides in the protein nanopore.

The above methods of Pacific Biosciences and Oxford Nanopore Technologies have yielded limited accuracy, with some improvement after combining data from multiple reads over the same sequence segments.
US2019/002971 describes nanopore arrays comprising opaque layers that reduce background fluorescence in optical signal collected in applications of such arrays for analyzing molecules. WO2018/195222 describes nanopore devices and methods of use. WO2017/180319 describes nanopore-based methods for analyzing polymers, such as polynucleotides or proteins, containing optical labels specific for different kinds of monomers.

There remains a strong need for new sequencing methods that are convenient to use, lower in cost, and provide high accuracy.

### BRIEF SUMMARY OF SELECTED EMBODIMENTS

the invention of claim 1 provides a method for determining a nucleotide sequence of a polynucleotide. The method comprises providing a solid state substrate comprising a cis side and a trans side, the substrate comprising a reaction well that defines a reaction volume and comprises (i) a proximal throughhole extending between the cis side and the trans side of the substrate, (ii) one or more side walls, and (iii) a distal opening. The solid state substrate further comprises an opaque metal layer that substantially blocks excitation light from penetrating into the reaction volume and from penetrating to the cis side of the substrate. Also provided is a carrier particle comprising a fluorescently labeled polynucleotide strand that is attached to the carrier particle. The fluorescently labeled polynucleotide strand comprises (i) a proximal end that is attached to the carrier particle, (ii) a distal end that is cleavable by an exonuclease, and (iii) at least one fluorescently labeled nucleotide comprising a fluorescent label. The carrier particle is located on the cis side of the substrate, but does not pass through the throughhole, such that the attached fluorescently labeled polynucleotide strand protrudes through the throughhole so that the distal end of the fluorescently labeled strand is in the reaction volume. The trans side of the substrate is illuminated with excitation light to create a fluorescence excitation zone adjacent to the distal opening of the reaction well. While the substrate is illuminated, the fluorescently labeled polynucleotide strand is reacted with an exonuclease so that mononucleotides are released serially from the distal end of the strand and diffuse out of the reaction volume through the distal opening, so that fluorescently labeled mononucleotides in the excitation zone emit fluorescent signals. The fluorescent signals are detected as a function of time, whereby a nucleotide sequence is determined from the time order of fluorescent signals detected from the released fluorescently labeled mononucleotides.

In some embodiments, the distal opening of the reaction well has a minimum diameter of at least 30 nm. In some embodiments, the distal opening of the reaction well has a minimum diameter of 50 nm to 150 nm.

In some embodiments, the one or more walls of the reaction well are not tapered. In some embodiments, the one or more walls of the reaction well are substantially cylindrical.

In some embodiments, the opaque metal layer comprises gold or aluminum. In some embodiments, the opaque metal layer has a thickness of 100 nm to 600 nm. In some embodiments, the solid state substrate comprises a plurality of opaque metal layers.

In some embodiments, the reaction well has a well depth of at least 200 nm. In some embodiments, the reaction well has a well depth of 200 nm to 1000 nm.

In some embodiments, the fluorescently labeled polynucleotide strand in the reaction volume comprises a fluorescently labeled polynucleotide segment containing at least 100 contiguous nucleotides.

In some embodiments, the throughhole has a minimum diameter of at least 2 nm. In some embodiments, the throughhole has a minimum diameter of 2 nm to 50 nm. In some embodiments, the substrate comprises a thin membrane layer that contains the proximal throughhole and that has a thickness of between 20 nm and 50 nm. In some embodiments, the thin membrane layer comprises silicon nitride.

In some embodiments, the excitation light has a wavelength of 380 nm or greater.

In some embodiments, the solid substrate comprises surface portion(s) that define the reaction volume, and the surface portion(s) comprise at least one surface passivation coating.

In some embodiments, one or more side walls of the reaction well comprise one or both of a silicon oxide coating and aluminum oxide coating.

In some embodiments, the fluorescently labeled polynucleotide strand comprises at least two different kinds of nucleotides, each kind labeled with a distinguishing fluorescent label.

In some embodiments, the carrier particle is not magnetic. In some embodiments, the carrier particle is magnetic.

In some embodiments, during said reacting, the carrier particle is maintained next to the proximal throughhole by a voltage bias.

In some embodiments, the carrier particle comprises a plurality of fluorescently labeled polynucleotide strands having polynucleotide sequences that are different from each other.

In some embodiments, after said reacting, the voltage bias is stopped to allow the carrier particle to move away from the proximal throughhole, so that the remaining fluorescently labeled polynucleotide strand is removed from the reaction volume, and then a voltage bias is applied to move the same or a different carrier particle toward the proximal throughhole so that a new fluorescently labeled polynucleotide strand is delivered into the reaction well for reacting with an exonuclease.

In some embodiments, the fluorescently labeled polynucleotide strand in the reaction volume comprises a double-stranded nucleic acid. In some embodiments, the fluorescently labeled polynucleotide strand in the reaction volume comprises a single-stranded nucleic acid. In some embodiments, the carrier particle comprises a plurality of fluorescently labeled polynucleotide strands, which in some embodiments, are single-stranded or double-stranded nucleic acids.

In some embodiments, the solid state substrate comprises a plurality of reaction wells. In some embodiments, the plurality of reaction wells are configured as a one-dimensional or two-dimensional array. In some embodiments, two or more of the plurality of reaction wells each contain a fluorescently labeled polynucleotide strand to be sequenced.

The above methods are also useful for determining nucleotide sequences of a plurality of polynucleotides.

The present disclosure also provides kits for use in methods of the invention.

### BRIEF DESCRIPTION OF THE DRAWINGS

Figures 1A-1D illustrate features and operation of an exemplary reaction well of a solid state substrate of the invention.
Figure 2 illustrates an exemplary sequencing apparatus of the invention.
Figure 3 illustrates an exemplary sequencing profile obtained from exonucleotytic cleavage of a fluorescently labeled polynucleotide strand.

### DETAILED DESCRIPTION OF EXEMPLARY EMBODIMENTS

While the invention is amenable to various modifications and alternative forms, specifics thereof are shown by way of example in the drawings and are described in further detail herein. It should be understood, however, that the intention is not to limit the invention to the particular embodiments described herein. On the contrary, the intention is to cover all modifications, equivalents, and alternatives falling within the spirit and scope of the invention.

For example, particular reaction well structures, particular labels, and fabrication examples are shown for purposes of illustration. It should be appreciated, however, that the disclosure is not intended to be limiting in this respect, as other structures, arrays of reaction wells, and other fabrication technologies that are not specifically detailed herein may be utilized to implement various aspects of the present inventions. Guidance for aspects of the invention is found in many references and treatises known to those with ordinary skill in the art, including, for example, Cao, Nanostructures & Nanomaterials (Imperial College Press, 2004); Levinson, Principles of Lithography, Second Edition (SPIE Press, 2005); Doering and Nishi, Editors, Handbook of Semiconductor Manufacturing Technology, Second Edition (CRC Press, 2007); Sawyer et al, Electrochemistry for Chemists, 2nd edition (Wiley Interscience, 1995); Bard and Faulkner, Electrochemical Methods: Fundamentals and Applications, 2nd edition (Wiley, 2000); Lakowicz, Principles of Fluorescence Spectroscopy, 3rd edition (Springer, 2006); Hermanson, Bioconjugate Techniques, Second Edition (Academic Press, 2008); and the like.

In some aspects, the invention is directed to fluorescence-based analysis of polynucleotides using sequential digestion of fluorescently labeled polynucleotide strands by exonuclease activity.

"Polynucleotide" and "oligonucleotide" are used interchangeably and mean a linear polymer of nucleotide monomers or analogs thereof. Nucleotide monomers in polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moieties, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is within the knowledge of one of ordinary skill, especially with guidance from treatises, such as Sambrook et al, Molecular Cloning, Second Edition (Cold Spring Harbor Laboratory, New York, 1989), and like references. Likewise, the oligonucleotide and polynucleotide may refer to either a single stranded form or a double stranded form (i.e. duplexes of an oligonucleotide or polynucleotide and its respective complement). It will be clear to one of ordinary skill which form or whether both forms are intended from the context of usage.

"Primer" means an oligonucleotide, either natural or synthetic, that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. However, much longer primers may be also used. Primers are employed in a variety of nucleic amplification reactions, for example, linear amplification reactions using a single primer, or in polymerase chain reactions, employing two or more primers. Guidance for selecting the lengths and sequences of primers for particular applications is known to those of ordinary skill in the art, as evidenced for example by Dieffenbach, editor, PCR Primer: A Laboratory Manual, 2nd Edition (Cold Spring Harbor Press, New York, 2003).

"Nucleic acid" encompasses polynucleotides and oligonucleotides as defined below, which may be natural or artificial, of any length, single-stranded, double-stranded, triple-stranded, linear, circular, or branched, and which are exemplified by 2'-deoxyribonucleic acid (DNA), ribonucleic acid (RNA) (such as ribosomal RNAs, transfer RNAs, microRNAs,), DNA/RNA hybrids, and DNA-RNA chimeras. Nucleic acids usually contain one or more standard nucleotide bases, such as adenine, cytosine, guanine, thymine, and uracil, and analogs thereof, such that the bases are usually capable of base-pairing with a base in an opposite base in a double strand form with complementary bases in a complementary strand.

"Sequence determination", "sequencing", "determining a nucleotide sequence", "determining a polynucleotide sequence", and similar terms, when referring to polynucleotides, includes the determination of partial or full nucleotide sequence information of one or more polynucleotides. These terms also include determining sequences of subsets of the full set of four natural nucleotides, A, C, G and T for DNA (or A, C, G or U for RNA), such as, for example, a sequence of only A's and C's of a target polynucleotide. These terms also include the determination of the identities, order, and locations of one, two, three or all of the four types of nucleotides within a target polynucleotide. In some embodiments, the terms include the determination of the identities, order, and locations of two, three or all of the four types of nucleotides within a target polynucleotide. In some embodiments sequence determination may be accomplished by identifying the order and locations of a single type of nucleotide, e.g. cytosines, within the target polynucleotide "catcgc ... " so that its sequence is represented as a binary code, e.g. "100101 ... " representing "c-(not c)(not c)c-(not c)-c . . . " and the like. In some embodiments, these terms may also include subsequences of a target polynucleotide that serve as a fingerprint for the target polynucleotide; that is, subsequences that uniquely identify a target polynucleotide, or a class of target polynucleotides, within a set of polynucleotides, e.g. all different RNA sequences expressed by a cell.

"Target polynucleotide" means a polynucleotide, or a segment of a polynucleotide, whose sequence is to be determined. If a target polynucleotide is double-stranded, then determination of a sequence of one strand of the target polynucleotide will reveal the sequence of the corresponding complementary strand, based on Watson and Crick base-pairing rules. If a target polynucleotide is single-stranded (or is intended to refer to a single strand in a double stranded nucleic acid), then determining a sequence of a strand that is complementary to the target polynucleotide (e.g., the sequence of a cDNA prepared by reverse transcription of a target RNA polynucleotide) will reveal the sequence of the target polynucleotide. Thus, depending on the context of how "target" is used, a target polynucleotide may be considered to be the a fluorescently labeled polynucleotide strand whose sequence is determined directly from a sequence of mononucleotides released serially by exonuclease-mediated digestion, or a target polynucleotide may be considered to be a polynucleotide strand whose sequence is complementary to a sequence of the fluorescently labeled polynucleotide strand.

"Target sequence" means a sequence of a target polynucleotide.

Polynucleotides may be obtained from any suitable sample source for sequencing using the present inventions. A wide variety of biological sources may be suitable, such as viruses, bacteria, mycobacteria, fungi, plants, animals, mammals, humans, etc. For complex organisms, such as humans, a variety of different sample types may be of interest, such as whole blood, plasma, serum, cells (such as red blood cells, white bloods cells, osteoclasts, osteoblasts, hepatocytes), urine, nasal mucus, feces, sputum, saliva, cerebral spinal fluid, mitochondria, exosomes, formalin fixed tissue samples, and tissue swabs.

Nucleic acids may be prepared and/or purified using any appropriate method. Many methods are known for different sample types. For example, many reagents and kits are commercially available for isolating DNA and RNA from various sample types, such as Qiagen, Roche, Promega, Biomerieux, etc. Nucleic acids may be isolated in their native state, or may be fragmented or modified in other ways prior to sequencing. For example, nucleic acids may be fragmented by mechanical methods (e.g., by sonication or nebulization) or by enzymatic digestion.

It is common to prepare plasmid libraries or other kinds of libraries for storing and/or preparing sample nucleic acids for subsequent use. Sample nucleic acids may be fragmented, or not fragmented, and may be capped by adaptors on both ends for easier manipulation. The adaptors may contain sequences that are useful for hybridizing primers to make complementary strands by primer extension, or to facilitate polymerase chain reaction amplification of sequences that are downstream of or between such adaptors.

For example, mechanically sheared DNA fragments usually require end-repair in which single stranded overhangs generated during fragmentation are filled-in using a DNA polymerase to make double-stranded blunt ended fragments. Some DNA polymerases that may be used for the fill-in reaction produce filled-in fragments having a non-templated 3'-A overhang, rather than a blunt end, which can increase the efficiency of adapter ligation. Prior to adapter ligation, the DNA fragments are size selected using commercial kits such as SPRI beads (Beckman Coulter Life Sciences) or gel purification. A double-stranded synthetic DNA adapter is then enzymatically ligated to both ends of the size-selected DNA fragments. The DNA adapters contain a blunt end on one side (or a 5'-T overhang for the corresponding 3'-A overhang fragments) through which they are ligated to the fragmented DNA. On the opposite end, a Y-shaped non-complementary single stranded portion ensures an oriented ligation of the adapter and provides priming sites for a primer extension, e.g., for incorporation of fluorescently labeled nucleotides by polymerase-mediated primer extension. See also Shendure & Ji, Nature Biotechnology 26(10): 1135-1145(2008).

Preferably, nucleic acids are purified to substantially remove non-target polynucleotides and other materials, such as cellular debris, proteins, etc. Methods for purifying nucleic acids from various sample types are well known and are described, for example, in publications such as Green and Sambrook, Molecular Cloning: A Laboratory Manual, Fourth Edition, CSHL Press (2012), Ausubel et al., Current Protocols in Molecular Biology, Wiley Press (2020), and the like.

Fluorescently labeled polynucleotide strands (also referred to as "fluorescently labeled strands") for sequencing in accordance with the present inventions may be prepared by any suitable method. Each fluorescently labeled strand may have a proximal end and a distal end. The proximal end is coupled, directly or indirectly, to a carrier particle as described further below. The distal end of the fluorescently labeled strand protrudes away from the carrier particle when the fluorescently labeled strand is coupled to a carrier particle. Each fluorescently labeled strand is capable of being cleaved by an exonuclease, so that mononucleotides, some or all of which comprise fluorescent labels, are released serially (one-by-one) from the distal end of the strand for subsequent detection (discussed further below).

In some embodiments, fluorescently labeled strands may be provided in single-stranded form, for serial cleavage of a distal end of the fluorescently labeled strand by a single-strand-specific exonuclease. In other embodiments, fluorescently labeled strands may be provided in double-stranded form comprising a fluorescently labeled strand, for serial cleavage of a distal end of the fluorescently labeled strand by a double-strand-specific exonuclease.

In some embodiments, a 5'-exonuclease is used. The 5'-exonuclease may be single-strand-specific or double-strand-specific. For a single-strand-specific 5'-exonuclease, the fluorescently labeled strand may be provided in single-stranded form such that the 3'-end is the proximal end coupled to the carrier particle, and the 5'-end is the distal end to be cleaved by the 5'-exonuclease. For a double-strand-specific 5'-exonuclease, all of the features of the immediately preceding sentence apply, except that the fluorescently labeled strand is provided in double-stranded form that comprises a complementary strand hybridized to the fluorescently labeled strand.

In some embodiments, a 3'-exonuclease is used. The 3'-exonuclease may be single-strand-specific or double-strand-specific. For a single-strand-specific 3'-exonuclease, the fluorescently labeled strand may be provided in single-stranded form such that the 5'-end is the proximal end coupled to the carrier particle, and the 3'-end is the distal end to be cleaved by the 3'-exonuclease. For a double-strand-specific 3'-exonuclease, all of the features of the immediately preceding sentence apply, except that the fluorescently labeled strand is provided in double-stranded form that comprises a complementary strand hybridized to the fluorescently labeled strand.

A fluorescently labeled strand may also be provided in a form comprising a double-stranded region and a single-stranded region. For example, if a fluorescently labeled strand is provided in a double-stranded form and is hybridized to a complementary strand such that the fluorescently labeled strand comprises a single-stranded 3'-overhang extending from a double-stranded region, the 3'-overhang can be cleaved by a single-strand specific 3'-exonuclease until the exonuclease reaches the double-stranded region. In this example, the 3'-end of the 3'-overhang is the distal end of the fluorescently labeled strand, and the 5'-end of the 3'-overhang is the proximal end of the fluorescently labeled strand. In an alternative example, if a fluorescently labeled strand is provided in a double-stranded form and is hybridized to a complementary strand that comprises a single-stranded 3'-overhang extending from a double-stranded region, a double-strand-specific 5'-exonuclease can cleave the recessed 5'-end of the fluorescently labeled strand in the double-stranded region. In this alternative example, the 5'-end of the fluorescently labeled strand is the distal end, and the 3'-end of the fluorescently labeled strand is the proximal end.

If a fluorescently labeled strand provided in a double-stranded form and is hybridized to a complementary strand such that the fluorescently labeled strand comprises a single-stranded 5'-overhang extending from a double-stranded region, the 5'-overhang can be cleaved by a single-strand specific 5'-exonuclease until the exonuclease reaches the double-stranded region. In this example, the 5'-end of the 5'-overhang is the distal end of the fluorescently labeled strand, and the 3'-end of the 5'-overhang is the proximal end of the fluorescently labeled strand. In an alternative example, if a fluorescently labeled strand is provided in a double-stranded form and is hybridized to a complementary strand that comprises a single-stranded 5'-overhang extending from a double-stranded region, a double-strand-specific 3'-exonuclease can cleave the recessed 3'-end of the fluorescently labeled strand in the double-stranded region. In this alternative example, the 3'-end of the fluorescently labeled strand is the distal end, and the 5'-end of the fluorescently labeled strand is the proximal end.

In some embodiments, the fluorescently labeled strands may be prepared by DNA polymerase-mediated extension (to make a fluorescently labeled DNA strand) or by RNA polymerase-mediated extension (to make a fluorescently labeled RNA strand) of a primer that is complementary to a sample polynucleotide sequence. For sequencing multiple sample sequences, a plurality of primers may be used. The primers may be all the same (i.e., a single primer having a selected primer sequence), or the primers may comprise a plurality of different primer sequences. In some embodiments, all primers have pre-selected primer sequences. In some embodiments, the primers comprise randomly generated primer sequences. In some embodiments, the primers may comprise all possible n-mer sequences, such as all possible 6-mer, 7-mer, 8-mer, 9-mer, or 10-mer sequences, which may facilitate priming of all possible sequences in a sample. In some embodiments, one or more primers may each comprise an affinity moiety to facilitate attachment of an extended primer to a carrier particle, as discussed further below.

In some embodiments, a nucleotide sequence of a target polynucleotide is determined by performing four separate reactions, each in a separate reaction well, in which copies of the target polynucleotide (or of its complementary sequence) have each of its four different kinds of nucleotides (A, C, G and T) labeled with a single fluorescent label.

In some embodiments, a fluorescently labeled polynucleotide strand comprises one kind of nucleotide that is labeled with a fluorescent label. In some embodiments, a fluorescently labeled polynucleotide strand comprises two different kinds of nucleotides, each kind labeled with a distinguishing fluorescent label. In some embodiments, a fluorescently labeled polynucleotide strand comprises at least two different kinds of nucleotides, each kind labeled with a distinguishing fluorescent label. In some embodiments, a fluorescently labeled polynucleotide strand comprises three different kinds of nucleotides, each kind labeled with a distinguishing fluorescent label. In some embodiments, a fluorescently labeled polynucleotide strand comprises at least three different kinds of nucleotides, each kind labeled with a distinguishing fluorescent label. In some embodiments, a fluorescently labeled polynucleotide strand comprises four different kinds of nucleotides, each kind labeled with a distinguishing fluorescent label.

In some embodiments, a nucleotide sequence of a target polynucleotide strand is determined by performing four separate reactions in which copies of the target polynucleotide strand have each of its four different kinds of nucleotide (A, C, G and T) labeled with one fluorescent label while at the same time the other nucleotides on the same target polynucleotide strand are labeled with a second fluorescent label. For example, if a first fluorescent label is attached to A's of the target polynucleotide strand in a first reaction, then a second fluorescent label is attached to C's, G's and T's (i.e. to the "not- A" nucleotides) of the target polynucleotide strands in the first reaction. Likewise, continuing with this example, in a second reaction, the first label is attached to C's of the target polynucleotide strand and the second fluorescent label is attached to A's, G's and T's (i.e. to the "not-C" nucleotides) of the target polynucleotide strand. And so on, for nucleotides G and T.

The same labeling scheme may be expressed in terms of conventional terminology for subsets of nucleotide types; thus, in the above example, in a first reaction, a first fluorescent label is attached to A's and a second fluorescent label is attached to B's; in a second reaction, a first fluorescent label is attached to C's and a second fluorescent label is attached to D's; in a third reaction, a first fluorescent label is attached to G's and a second fluorescent label is attached to H's; and in a fourth reaction, a first fluorescent label is attached to T's and a second fluorescent label is attached to Vs.

In some embodiments, a fluorescently labeled polynucleotide strand comprises a single fluorescent label attached to a single kind of monomer, for example, every T (or substantially every T) of a polynucleotide strand is labeled with a fluorescent label, e.g. a cyanine dye. In such embodiments, a collection, or sequence, of fluorescent signals from the polynucleotide strand may form a signature or fingerprint for the particular polynucleotide. In some such embodiments, such fingerprints may or may not provide enough information for a sequence of monomers to be determined.

In some embodiments, fluorescent labels are mutually quenching. In some embodiments, fluorescent labels are not mutually quenching.

Mutually quenching fluorescent labels have the following properties: (i) each member quenches fluorescence of every member (for example, by FRET or by static or contact mechanisms), and (ii) each member generates a distinct fluorescent signal when excited and when in a non-quenched state. That is, if a mutually quenching set consists of two dyes, D1 and D2, then (i) D1 is self-quenched (e.g. by contact quenching with another D1 molecule) and it is quenched by D2 (e.g. by contact quenching) and (ii) D2 is self-quenched (e.g. by contact quenching with another D2 molecule) and it is quenched by D1 (e.g. by contact quenching). Contact quenching often occurs strongly between fluorescent labels on adjacent mononucleotides, but contact quenching may also occur between fluorescent labels on mononucleotides that are not adjacent to each other. Benefits of using mutually quenching fluorescent labels include, for example, minimizing non-specific fluorescence background from fluorescently labeled polynucleotide strands, and preserving the fluorescent labels from adventitious radical and triplet state species that may exist even in the non-illuminated (or negligibly illuminated) regions of reaction wells.

Fluorescent labels include any fluorescent dyes chosen by the user for identifying attached mononucleotides in the methods of the invention. Exemplary fluorescent labels for labeling NTPs, dNTPs, NTP analogs and dNTP analogs, include, but are not limited to, xanthenes, fluoresceins, rhodamines, sulforhodamines, rhodals, cyanines, coumarins, and pyrenes. If different fluorescent labels are used to identify and distinguish different kinds of nucleotides, then the fluorescent labels can be from the same structural class of fluorescent labels (e.g., all are fluoresceins) or from different classes of fluorescent labels.

Exemplary guidance for selecting fluorescent labels for mutually quenching sets may be found in the following references: Johansson, Methods in Molecular Biology, 335: 17-29 (2006); Marras et al, Nucleic Acids Research, 30: el22 (2002); and the like. In some embodiments, members of a mutually quenching set comprise organic fluorescent dyes that comprise components or moieties capable of stacking interactions, such as aromatic ring structures. In some embodiments, exemplary mutually quenching sets of fluorescent labels may be selected from rhodamine dyes, fluorescein dyes and cyanine dyes. In some embodiments, a mutually quenching set may comprise a rhodamine dye, TAMRA, and a fluorescein dye, FAM. In some embodiments, mutually quenching sets of fluorescent dyes may comprise two or more dyes selected from Oregon Green 488, Fluorescein-EX, fluorescein isothiocyanate, Rhodamine Red-X, Lissamine rhodamine B, Calcein, fluorescein, rhodamine, one or more BODIPY dyes, Texas Red, Oregon Green 514, and one or more Alexa Fluors. Exemplary BODIPY dyes include BODIPY FL, BODIPY R6G, BODIPY TMR, BODIPY 581/591, BODIPY TR, BODIPY 630/650 and BODIPY 650/665. Exemplary Alexa fluorescent labels include Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750 and Alexa Fluor 790.

In further embodiments, exemplary fluorescent labels for labeling NTPs, dNTPs, NTP analogs and dNTP analogs, include, but are not limited to, Alexa 488, AMCA, Atto 655, Cy3, Cy5, Evoblue 30, fluorescein, Gnothis blue 1, Gnothis blue 2, Gnothis blue 3, Dy630, Dy635, MR121, rhodamine, Rhodamine Green, Oregon Green, TAMRA, and the like. Exemplary fluorescent labels for dUTP analogs include, but are not limited to, Alexa 488, AMCA, Atto 655, Cy3, Cy5, Dy630, Dy665, Evoblue 30, Evoblue 90, fluorescein, Gnothis blue 1, Gnothis blue 2, Gnothis blue 3, MR121, Oregon Green, rhodamine, Rhodamine Green, TAMRA, and the like. Exemplary fluorescent labels for dCTP analogs include, but are not limited to, Atto 655, Cy5, Evoblue 30, Gnothis blue 3, rhodamine, Rhodamine Green, TAMRA, and the like. Exemplary fluorescent labels for dATP analogs include, but are not limited to, Atto 655, Cy5, Evoblue 30, Gnothis blue 3, Rhodamine Green, and the like. Exemplary fluorescent labels for dGTP analogs include, but are not limited to, Evoblue 30, Gnothis blue 3, Rhodamine Green, and the like. Exemplary pairs of fluorescent labels for dUTP analogs and dCTP analogs include, but are not limited to, TAMRA, Rhodamine Green, Atto 655, Evoblue 30, Evoblue 30, Atto 655, Evoblue 30, Gnothis blue 3, Evoblue 30, Rhodamine Green, Gnothis blue 1, Rhodamine Green, Gnothis blue 2, Atto 655), Gnothis blue 3, Cy5, and the like.

In further embodiments, exemplary fluorescent labels for labeling NTPs, dNTPs, NTP analogs and dNTP analogs, include, but are not limited to, Oregon Green 488, fluorescein-EX, FITC, Rhodamine Red-X, Lissamine rhodamine B, calcein, fluorescein, rhodamine, BODIPYs, and Texas Red, e.g. which are disclosed in Molecular Probes Handbook -A Guide to Fluorescent Probes and Labeling Technologies, 11th Edition (2010) as revised online as of the date of the present disclosure.

Further guidance for selecting kinds of nucleotide to label, kinds of labels, linkers for attachment, and nucleic acid polymerases for synthesizing fluorescently labeled polynucleotide strands in the presence of NTPs, dNTPs, NTP analogs, and/or dNTP analogs, can be found in the following references: Goodman et al, U.S. patent 5,945,312; Jett et al, U.S. patent 5,405,747; Muehlegger et al, U.S. patent publication US2004/0214221 ; Giller et al, Nucleic Acids Research, 31(10): 2630-2635 (2003); Tasara et al, Nucleic Acids Research 31(10): 2636-2646 (2003); Augustin et al, J. Biotechnology, 86: 289-301 (2001); Brakmann, Current Pharmaceutical Biotechnology, 5(1): 119-126 (2004); and Anderson et al., BioTechniques 38:257-264 (2005); for example.

Exemplary nucleic acid polymerases for synthesizing fluorescently labeled polynucleotide strands in the presence of NTPs, dNTPs, NTP analogs and/or dNTP analogs, include, but are not limited to, Vent (exo minus) polymerase, Taq polymerase, E. coli Pol I, Tgo (exo minus) polymerase, Klenow fragment (exo minus), Thermococcus kodakaraensis KOD1 DNA polymerase (EMD Millipore and other suppliers), Deep Vent (exo minus) polymerase, Therminator polymerase, Pfu (exo minus) polymerase, Pfu alfalfa mosaic virus reverse transcriptase, murine leukemia virus reverse transcriptase, T4 polymerase, and the like. In some embodiments, exemplary nucleic acid polymerases include, but are not limited to, Vent (exo minus) polymerase and Klenow fragment (exo minus). It is noted that exo minus polymerases have greater processivity relative to corresponding polymerases that retain 3'-exonuclease activity, so that synthesis of a fluorescently labeled polynucleotide strands can be accomplished more efficiently.

In other embodiments, selected kinds of nucleotides of a strand are labeled by incorporating analog dNTPs of the selected kinds of nucleotides in an extension reaction, wherein the analog dNTPs are derivatized with orthogonally reactive functionalities that allow attachment of different labels to different kinds of nucleotides in a subsequent reaction, as described, for example, in Jett et al, U.S. patent 5,405,747.

The carrier particles have dimensions that are sufficiently large to prevent the carrier particles from moving through the throughholes of the reaction wells into the reaction wells. Each carrier particle is capable of being moved by an electromagnetic force to be near a throughhole of a reaction well to deliver the distal end of a first fluorescently labeled polynucleotide strand, which is attached to the carrier particle, through the throughhole into a reaction well. In some embodiments, the carrier particles are magnetic carrier particles. In some embodiments, the carrier particles are not magnetic carrier particles. In some embodiments, the electromagnetic force is a voltage bias. In some embodiments, the electromagnetic force is a magnetic force.

In some embodiments, carrier particles have a diameter, or a largest diameter, that is at least 15 nm, or at least 20 nm, or at least 25 nm, or at least 30 nm, or from 15 nm to 100 nm, or from 15 nm to 75 nm, or from 15 nm to 50 nm, or from 20 nm to 50 nm. However, carrier particles having larger or smaller diameters may also be used. In some embodiments, carrier particles of the invention are or comprise one or a plurality of nanoparticles. As used herein, "nanoparticle" refers to a carrier particle having a diameter, or largest diameter, that is less than 200 nm, or less than 150 nm, or less than 100 nm.

The carrier particles may be charged or uncharged. The carrier particles may have a net neutral charge, a net positive charge, or a net negative charge, based on the net balance of positively charged groups and negatively charged groups on the particles under the pH conditions of the surrounding aqueous medium, which usually comprises an aqueous buffer. Preferably, for control of movement by an electric field (voltage bias), the carrier particles have a net negative charge when they comprise one or more attached fluorescently labeled polynucleotide strands.

In some embodiments, each carrier particle is capable of being moved by an electromagnetic force away from a throughhole of a reaction well, to remove a cleaved fluorescently labeled polynucleotide strand from the throughhole. In preferred embodiments of methods of the present invention, after exonuclease cleavage of a fluorescently labeled polynucleotide strand in a well, the carrier particle is moved away from the well so that the cleaved strand is withdrawn from the well, and then the same or a different carrier particle is moved near the throughhole of the well to deliver the distal end of a second (sometimes called "new") fluorescently labeled polynucleotide strand through the throughhole into the reaction well. To facilitate movement of distal ends of fluorescently labeled polynucleotide strand into and out of the reaction wells by voltage bias or magnetic field, the carrier particles are not covalently coupled to the throughholes.

In some embodiments, the carrier particles comprise spherical particles. In some embodiments, the carrier particles comprise non-spherical particles, having, for example, elliptoid or irregular shapes. In some embodiments, the carrier particles comprise both spherical particles and non-spherical particles. In some embodiments, the carrier particles are provided as a uniform population of substantially identical carrier particles within a size range (e.g., within plus or minus a standard deviation or coefficient of variation), but the carrier particles are not necessarily identical, provided that they effectively carry and deliver fluorescently labeled polynucleotide strands to the reaction wells.

Carrier particles may be made from any materials that are suitable for the purposes of the present invention. In some embodiments, the carrier particles are metal particles, such as metal nanoparticles. In some embodiments, the carrier particles are gold nanoparticles. In some embodiments, the carrier particles are silver nanoparticles. In some embodiments, one or more carrier particles comprise one or more magnetic materials, such as iron or iron oxide, that allow the particles to be moved by a magnetic field. In some embodiments, the carrier particles are iron oxide particles. In some embodiments, the carrier particles are silica particles or controlled pore glass particles.

In some embodiments, the carrier particles comprise an immobilized protein, such as streptavidin or an antigen-specific antibody, for binding a biotin moiety or antigen moiety that is attached to or associated with a fluorescently labeled polynucleotide strand to be sequenced. In some embodiments, the carrier particles are proteins, such as streptavidins or antibodies, for binding one or more biotinylated oligonucleotides or antigen-oligonucleotide conjugates.

Fluorescently labeled polynucleotide strands may be attached to carrier particles by any suitable means. Usually, fluorescently labeled polynucleotide strands are attached to carrier particles by means of a capture moiety. Capture moieties usually comprise members of a pair of moieties that have a mutual affinity for each other (also referred to as a "binding pair"). In some embodiments, capture moieties are polypeptides such as antibodies or antigens. In some embodiments, capture moieties are oligonucleotides that are complementary to sequences in, or associated with, the fluorescently labeled polynucleotide strand to be sequenced.

Capture moieties may be monovalent (for capturing one binding partner) or multivalent (for capturing multiple binding partners). Avidin is an example of a monovalent capture moiety, and streptavidin, with four biotin binding sites, is an example of a multivalent capture moiety. In some embodiments, the capture moiety comprises an antibody (for specifically binding one or two antigens). More generally, the capture member may be any member of a binding pair for which the other member of the pair is associated with a fluorescently labeled polynucleotide strand to facilitate attachment of the fluorescently labeled polynucleotide strand to the carrier particle.

Usually, if a carrier particle is not itself a capture moiety (e.g., if the carrier particle is not streptavidin, an antibody, or another monovalent or multivalent entity), the carrier particle comprises at least one, and preferably a plurality, of capture moieties by which fluorescently labeled polynucleotide strands may be attached directly or indirectly to the carrier particles.

Capture moieties are usually attached to carrier particles by linkers. Any suitable linker may be used. Since exonuclease cleavage and other elements of the present invention are usually performed in aqueous solution, linkers are usually hydrophilic. Exemplary linkers include polymers such as polyethylene glycol, polyamides, poly(polyethylene glycol phosphates), polyalkyl phosphates, polyamines, and the like. Such linkers may have any suitable length. Some illustrative linkers and conjugation methods are described in Example 1 below.

Exemplary functional group pairs and their resulting linkages for attaching capture moieties to carrier particles are shown in Table 1 below.

**Table 1**

| **Carrier Particle** | **Oligonucleotide** | **Linkage** |
|---|---|---|
| amino | NHS (N-hydroxy succinimide) | amide -NH-C(=O)- |
| amino | carboxyl | amide -NH-C(=O)- |
| carboxyl | amino | amide -C(=O)NH- |
| thiol | thiol | disulfide-S-S- |
| gold | thiol | gold thiolate Au-S- |
| azide | DBCO or BCN | cycloaddition adduct |
| maleimide | thiol | Michael adduct |

Fluorescently labeled polynucleotide strands are immobilized on (attached to) carrier particles by any suitable means. In some embodiments, fluorescently labeled polynucleotide strands are each synthesized by nucleic acid polymerase-mediated template-dependent primer extension using sample template strands. In some embodiments, a fluorescently labeled polynucleotide strand is synthesized from a sample template nucleic acid strand in solution, before attachment of the fluorescently labeled polynucleotide strand to a carrier particle. In some embodiments, fluorescently labeled polynucleotide strands are synthesized on the carrier particle by polymerase-mediated primer extension after a sample template nucleic acid strand has been hybridized to a complementary primer (acting as a "capture moiety") attached to the carrier particle.

In some embodiments, the carrier particle comprises one or more oligonucleotides that are complementary to a sequence of a sample nucleic acid strand. In some embodiments, the carrier particle comprises one or more oligonucleotides that are complementary to a sequence of a fluorescently labeled polynucleotide strand. In some embodiments, the one or more oligonucleotides are coupled by their 5'-ends to the carrier particle. In some embodiments, the one or more oligonucleotides are coupled by their 3'-ends to the carrier particle.

In some embodiments, when, for example, an oligonucleotide is coupled by its 5'- end to a carrier particle, a fluorescently labeled polynucleotide strands may be synthesized on the carrier particle by polymerase-mediated template-dependent extension of the 3' end (the 3'-hydroxyl) of the oligonucleotide as a primer. A sample nucleic acid is hybridized to the immobilized oligonucleotide such that the 3'-end of the oligonucleotide is hybridized to a sequence region that is upstream of the region of the sample nucleic acid strand to be sequenced. In the presence of one or more different nucleotide triphosphates that comprise fluorescently labeled nucleotide triphosphates corresponding to one or more of ATP, CTP, GTP, and TTP or UTP, the polymerase synthesizes a fluorescently labeled polynucleotide strand that is complementary to the hybridized sample nucleic acid strand by extending the 3'- end of the immobilized oligonucleotide as a primer to incorporate complementary labeled nucleotides into a growing primer extension product that becomes the fluorescently labeled strand.

In some embodiments, as noted above, the fluorescently labeled polynucleotide strands may be formed in solution using a non-immobilized primer. The primer is hybridized to a complementary sample nucleic acid sequence that is upstream of the region of the sample nucleic acid strand to be sequenced. After polymerase-mediated primer extension in the presence of one or more different fluorescently labeled nucleotide triphosphates, the resulting hybridization complex (which comprises the sample nucleic acid strand hybridized to the newly synthesized fluorescently labeled polynucleotide strand) may be contacted with a capture oligonucleotide that is already immobilized on the carrier particle, so that the fluorescently labeled polynucleotide strand becomes immobilized on the carrier particle by hybridization of the sample strand / fluorescently labeled polynucleotide strand complex to the capture oligonucleotide.

In some embodiments, after a fluorescently labeled polynucleotide strand is formed in solution using a non-immobilized primer, the resulting sample strand / fluorescently labeled polynucleotide strand hybridization complex is immobilized on a carrier particle by hybridization of the capture oligonucleotide to a sequence in the sample nucleic acid strand.

In some embodiments, after a fluorescently labeled polynucleotide strand is formed in solution using a non-immobilized primer, the resulting sample strand / fluorescently labeled polynucleotide strand hybridization complex is immobilized on a carrier particle by hybridization of the capture oligonucleotide to a sequence in the fluorescently labeled polynucleotide strand. For example, the sample strand / fluorescently labeled polynucleotide strand hybridization complex may be immobilized on a carrier particle by hybridization of the capture oligonucleotide to a sequence in the primer that was used to synthesize the fluorescently labeled polynucleotide strand.

In some embodiments, the primer comprises a non-polynucleotide affinity moiety, such biotin or an antigen moiety, and the carrier particle comprises a streptavidin moiety or antibody moiety, so that after a fluorescently labeled polynucleotide strand is formed in solution using a non-immobilized primer, the resulting sample strand / fluorescently labeled polynucleotide strand hybridization complex is immobilized on the carrier particle by binding between the immobilized streptavidin moiety and the biotin moiety (or between the immobilized antibody and the antigen moiety).

As noted above, fluorescently labeled polynucleotide strands of the invention are cleaved using one or more exonucleases. An exonuclease is selected to have substantially no endonuclease activity, to ensure that the exonuclease cleaves only single, consecutive mononucleotides from the distal end of the fluorescently labeled strand. The exonuclease may be a 3'-specific exonuclease or a 5'-specific exonuclease, and the exonuclease may cleave a fluorescently labeled strand that is provided in single-stranded or double-stranded form.

Any suitable exonuclease may be used. Exonucleases may be native (i.e., have a chemical structure found in nature) or modified relative to their native structures, and may be from their natural sources or from recombinant hosts. For example, exonucleases may be chemically modified after isolation or purification, and may also be generated by combinatorial and recombinant techniques, including for example screening for exonucleases with desired properties. Exemplary 3'-specific exonucleases include, for example, E. coli exonuclease III, exonuclease X, yeast TatD exonuclease XI, the 3'-exonuclease activity of T7 DNA polymerase, the 3'-exonuclease activity of T4 DNA polymerase, the 3'-exonuclease activity E. coli DNA polymerase I Klenow fragment, the 3'-exonuclease activity of Vent^{®} DNA polymerase, the 3'-exonuclease activity of Deep Vent^{®} DNA polymerase, the 3'-exonuclease activity of Q5^{®} High-Fidelity DNA polymerase, the 3'-exonuclease activity of Q5U^{®} Hot Start High-Fidelity DNA polymerase, the 3'-exonuclease activity of Phusion^{®} High-Fidelity DNA polymerase, and the 3'-exonuclease activity of phi29 DNA polymerase. Exemplary 5'-specific exonucleases include, for example, T7 exonuclease, the 5'-exonuclease activity of Taq DNA polymerase, the 5'-exonuclease activity of Epimark^{®} Hot Start Taq DNA polymerase, and the 5'-exonuclease activity of Bst DNA polymerase.

In some cases, an exonuclease may cleave a strand that is present as a single strand or is present in a double stranded structure. For example, yeast TatD exonuclease XI is able to cleave a nucleic acid strand in the 3' to 5' direction in a single-stranded form or a double-stranded form.

Nucleases that have both 3'-exonuclease activity and 5'-exonuclease activity include, for example, E. coli DNA polymerase I, OneTaq^{®} DNA polymerase, and LongAmp^{®} Taq DNA polymerase. Such exonucleases may be used without modification to exploit one (e.g., 3' to 5') of their exonuclease activities to cleave fluorescently labeled polynucleotide strands if none of the polynucleotide strands in the reaction mixture are cleavable by the other (e.g., 5' to 3') exonuclease activity. Alternatively, it may be desirable to modify such exonucleases, e.g., by recombinant or chemical modification, to retain the desired exonuclease activity and inactivate the unwanted exonuclease activity.

Exonucleases that have exonuclease activity towards single-stranded nucleic acids, include, for example, E. coli RNAse T, E. coli exonuclease I, E. coli thermolabile exonuclease I, E. coli exonuclease VII, mung bean nuclease, venom exonuclease, exonuclease VII, RecJf exonuclease, and BAL-31 exonuclease.

To the extent that any of the foregoing enzymes (e.g., polymerases) have other enzymatic activities in addition to exonuclease activity, reaction conditions are selected to suppress those activities so that they do not interfere with polynucleotide sequencing according to the present invention. For example, in some embodiments, polymerization activity will be negligible or non-existent under exonuclease cleavage reaction conditions that do not include any of the standard nucleotide 5-triphosphates that are substrates for polymerization.

In some aspects of the inventions disclosed herein, a solid state substrate comprises a cis side and a trans side. The substrate comprises a reaction well that defines a reaction volume. The reaction well comprises (i) a proximal throughhole extending between the cis side and the trans side of the substrate, (ii) one or more side walls, and (iii) a distal opening. The solid state substrate further comprises an opaque metal layer that substantially blocks excitation light that is incident on the trans side of the substrate from penetrating into the reaction volume of the reaction well and from penetrating to the cis side of the substrate.

Reaction wells for containing fluorescently labeled polynucleotide strands to be sequenced may have any of a variety of shapes and sizes. For example, although cylindrical wells with circular cross-sections and parallel side walls are suitable, reaction wells may also have elliptical, triangular, square, rectangular, pentagonal, hexagonal, octagonal or other regular or irregular cross-sectional shapes, with parallel or non-parallel side walls. For example, the side walls of reaction wells having any of the foregoing shapes may be parallel, tapered, truncated-conical, or hour-glass shaped. For example, a cylindrical well may be considered to have a single side wall that is inherently parallel with itself.

Reaction wells may have any of a variety of dimensions that may be chosen by the user. The choice of specific dimensions can take into consideration a selected length and a minimum diameter of the fluorescently labeled strands that will be sequenced, whether the fluorescently labeled strands are in single- or double-stranded form, and any other relevant considerations.

The depth and minimum diameters of reaction wells are usually selected so that each reaction well can contain (1) a distal end of a fluorescently labeled polynucleotide strand to be sequenced and also (2) an exonuclease molecule that is bound to the distal end of the strand during nucleolytic cleavage of terminal mononucleotides.

As used herein, "minimum diameter" means the shortest diameter of a reaction well or of a throughhole, as applicable. For example, a cylinder has a single diameter, which is the minimum diameter. For a reaction well having a square overhead cross-section that is perpendicular to the depth axis of the reaction well, the minimum diameter is the distance between (and perpendicular to) two opposing walls of the reaction well (the length of a side of the square cross-section), whereas the maximum diameter is the length of a diagonal across the square cross-section. For a reaction well having tapered or other non-parallel walls, the minimum diameter of the reaction well is the shortest dimension in a cross-section of the well. More generally, the distal opening, and at least a portion of the reaction well extending from the distal opening, have a minimum diameter that satisfies requirements (1) and (2) above. Therefore, if the distal opening of a reaction well has a particular minimum diameter, then the minimum diameter of at least a portion of, or all of, the reaction well extending from the distal opening towards the throughhole of the well is equal to or greater than the minimum diameter of the distal opening of a reaction well. In some embodiments, the proximal throughhole may have substantially the same diameter as the diameter of the distal opening and of the reaction volume defined by the side wall(s).

In some embodiments, the reaction well, or the distal opening of the reaction well, has a minimum diameter of at least 30 nm, or at least 40 nm, or at least 50 nm, or at least 60 nm, or at least 70 nm, or at least 80 nm, or at least 90 nm, or at least 100 nm. In some embodiments, the reaction well has a minimum diameter that is less than 150 nm, or less than 120 nm, or less than 100 nm, or less than 90 nm, or less than 80 nm. In some embodiments, the reaction well has a minimum diameter of 30 nm to 250 nm, or 30 nm to 150 nm, or 30 nm to 120, or 30 nm to 100, or 30 nm to 90 nm, or 50 nm to 150 nm, or 50 nm to 120 nm, or 50 nm to 100 nm, or 50 nm to 90 nm, or 80 nm to 120 nm.

The depth of the reaction well is usually selected to be longer than the length of the segment of a fluorescently labeled polynucleotide strand that will be cleaved by the exonuclease to generate sequence information. Double-stranded DNA has a relatively rigid rod shape, with unit length of about one basepair per 3.6 Angstroms (0.36 nm). Therefore, a dsDNA segment that contains 1000 contiguous basepairs has a length of about 360 nm. Accordingly, a reaction well depth of 400 nm may be appropriate to enclose an immobilized 1000 bp dsDNA without exposing the distal end of the dsDNA to unwanted excitation light, and a well depth of 500 nm may provide even better protection from unwanted excitation light. Alternatively, for a single-stranded DNA or RNA strand, a reaction well depth of 400 nm may enclose an immobilized fluorescently labeled strand having more than 1000 contiguous bases without exposing the distal end of the dsDNA to unwanted excitation light, since single-stranded nucleic acids are less rigid and have a smaller diameter than double-stranded nucleic acids.

In some embodiments, the reaction well has a well depth of at least 150 nm, or at least 200 nm, or at least 300 nm, or at least 400 nm, or at least 500 nm. In some embodiments, the reaction well has a well depth that is less than 1000 nm, or is less than 800 nm, or is less than 700 nm, or is less than 600 nm, or is less than 500 nm. In some embodiments, the reaction well has a well depth of 150 nm to 1000 nm, or 150 nm to 800 nm, or 150 nm to 700 nm, or 150 nm to 600 nm, or 150 nm to 500 nm. In some embodiments, the reaction well has a well depth of 200 nm to 1000 nm, or 200 nm to 800 nm, or 200 nm to 700 nm, or 200 nm to 600 nm, or 200 nm to 500 nm. In some embodiments, the reaction well has a well depth of 300 nm to 1000 nm, or 300 nm to 800 nm, or 300 nm to 700 nm, or 300 nm to 600 nm, or 300 nm to 500 nm. In some embodiments, the reaction well has a well depth of 400 nm to 1000 nm, or 400 nm to 800 nm, or 400 nm to 700 nm, or 400 nm to 600 nm, or 400 nm to 500 nm.

As noted above, the reaction well also comprises a proximal throughhole extending between the cis side and the trans side of the substrate. Here, "proximal" throughhole means that the throughhole is closer to the cis side of the substrate than is the distal opening of the associated reaction well. Each proximal throughhole has a minimum diameter that is (1) sufficiently large to allow the distal end of a fluorescently labeled polynucleotide strand to be drawn into and through the throughhole by an electric field, and (2) is sufficiently small to prevent the carrier particle to which the fluorescently labeled strand is attached from passing through the throughhole to the trans side of the substrate. Preferably, the minimum diameter of the proximal throughhole is sufficiently small to prevent any exonuclease molecules from passing through the proximal throughhole from the trans side to the cis side of the substrate. In some embodiments, each proximal throughhole has a minimum diameter that is smaller than the smallest dimension of the exonuclease.

In some embodiments, the proximal throughhole has a minimum diameter of at least 2 nm. In some embodiments, the proximal throughhole has a minimum diameter of 2 nm to 50 nm, or 3 nm to 50 nm, or 5 nm to 50 nm, or 10 nm to 50 nm, or 20 nm to 50 nm, or 2 nm to 40 nm, or 3 nm to 40 nm, or 5 nm to 40 nm, or 10 nm to 40 nm, or 20 nm to 40 nm, or 3 nm to 30 nm, or 5 nm to 30 nm, or 10 nm to 30 nm, or 2 nm to 20 nm, or 3 nm to 20 nm, or 5 nm to 20 nm, or 10 nm to 20 nm, or 2 nm to 10 nm, or 3 nm to 10 nm, or 5 nm to 10 nm.

In some embodiments, the proximal throughhole has a longitudinal thickness of at least 10 nm, or at least 15 nm, or at least 20 nm, or from 10 to 60 nm, or from 10 to 50 nm or from 10 to 40 nm, or from 20 to 60 nm, or from 20 to 50 nm, or from 20 to 40 nm, or from 30 to 60 nm, or from 30 to 50 nm.

In some embodiments, the substrate comprises a thin membrane layer that contains the proximal throughhole. In some embodiments, the thin membrane layer comprises silicon nitride (SiN).

Substrates comprising reaction wells for use in the present inventions may be fabricated by any suitable method, in various forms of solid materials including but not being limited to silicones (e.g. Si₃N₄, SiO₂), metals, metal oxides (e.g. Al₂O₃) plastics, glass, semiconductor material, and combinations thereof. Fabrication techniques for making solid state substrates can be found in the following exemplary references: Golovchenko et al, U.S. patent 6,464,842; Sauer et al, U.S. patent 7,001,792; Su et al, U.S. patent 7,744,816; Meller et al, International patent publication WO2009/020682; Yan et al, Nano Letters, 5(6): 1129-1134 (2005); Wanunu et al, Nano Letters, 7(6): 1580-1585 (2007); Dekker, Nature Nanotechnology, 2: 209-215 (2007); Storm et al, Nature Materials, 2: 537-540 (2003); Zhe et al, J. Micromech. Microeng., 17: 304-313 (2007); and the like.

The solid state substrate comprises one or more light-blocking layers referred to herein as opaque metal layers. Each opaque metal layer reflects and/or absorbs incident light from the excitation beam, thereby (1) protecting the fluorescently labeled strands in the reaction well and on the cis side of the substrate from photobleaching and from other damage caused by incident light, and (2) preventing incident light from causing labels in the fluorescently labeled strands to fluoresce before being cleaved from the labeled strand by the exonuclease, potentially interfering with the correct fluorescent signals from cleaved fluorescently labeled mononucleotides.

An opaque metal layer may comprise Sn, Al, V, Ti, Ni, Mo, Ta, W, Au, Ag or Cu, for example, and/or alloys or combinations thereof. In some embodiments, an opaque metal layer comprises Al, Au, Ag or Cu. In some embodiments, an opaque metal layer comprises aluminum (Al) or gold (Au). The composition of the opaque metal layer may be selected based on the wavelength-dependence of the metal's reflectance of incident light. For fluorescence detection in the present invention, incident light is typically in the visible spectrum in the range of from about 380 nm to about 740 nm. Aluminum exhibits a reflectance of about 90% across the entire visible spectrum, making it a good candidate for use as an opaque layer. Gold exhibits a reflectance of about 35% for wavelengths between about 260 nm to about 480 nm and then rises sharply for wavelengths between about 480 nm to 700, exceeding about 90% for wavelengths greater than about 550 nm. Thus, gold has good light blocking characteristics across the visible spectrum, especially for wavelengths above about 480 nm, particularly in the red and infrared regions. Silver has a reflectance above about 80% for wavelengths above about 350 nm.

In some embodiments, the substrate comprises two or more opaque metal layers. For example, the substrate may comprise a gold layer and an aluminum layer, both of which reflect and/or absorb incident light impinging on the trans side of the substrate. In some embodiments, the substrate comprises a gold layer over an aluminum layer, such that the aluminum layer is closer to the cis side of the substrate than is the gold layer. One benefit of having a gold top layer (which may also be referred to as an outer gold layer or distal gold layer, and which surrounds the distal opening of the reaction well) is that gold can enhance the intensity of light excitation around the distal opening of the reaction well, thereby increasing the yield of fluorescent signals from each released fluorescently labeled mononucleotide that diffuses through the excitation zone. In some embodiments, the substrate comprises a distal aluminum layer over a gold layer, such that the gold layer is closer to the cis side of the substrate than is the aluminum layer.

The thickness of an opaque metal layer may vary and depends on the physical and chemical properties of material composing the opaque layer. In some embodiments, the thickness of an opaque layer may be at least 40 nm, or at least 80 nm, or at least 120 nm, or at least 200 nm, or at least 300 nm. In other embodiments, the thickness of an opaque layer may be in the range of from 50 to 700 nm; in other embodiments, the thickness of an opaque layer may be in the range of from 100 to 600 nm. If the substrate comprises more than one opaque metal layer, then "thickness" refers to the thickness of each individual layer.

An opaque metal layer need not block (i.e. reflect or absorb) 100 percent of the light from an excitation beam. In some embodiments, the opaque metal layer, or plurality of opaque metal layers if more than one layer is present, blocks at least 30%, at least 50%, or at least 90%, or at least 95%, or at least 99%, or at least 99.5%, or at least 99.9% of the excitation light that is incident on the distal opening of a reaction well at a depth that is 50 nm from the proximal throughhole of the reaction well.

Opaque layers may be fabricated by a variety of techniques. Material deposition techniques may be used including chemical vapor deposition, electrodeposition, epitaxy, thermal oxidation, physical vapor deposition, including evaporation and sputtering, casting, for example. In some embodiments, atomic layer deposition may be used, e.g. U.S. patent 6,464,842; Wei et al, Small, 6(13): 1406-1414 (2010),

The solid state substrate may comprise other layers. For example, a solid state substrate may comprise one or more non-opaque layers to increase the depth of the reaction well(s) in the substrate. Such a dielectric layer may comprise SiO₂, TiO₂, for example.

In some embodiments, a solid state substrate may comprise a thin adhesive layer between two other layers to enhance stability of the layers and deter delamination or other kinds of damage. For example, including a thin adhesive layer between a gold layer and an aluminum layer can enhance the adherence of the gold layer in the solid state substrate, as taught by Aouani et al., ACS Nano 3(7):2043-2048 (2009). Such a thin adhesive layer may comprise any suitable material, including for example chromium, titanium, titanium dioxide, chromium oxide, or nickel. A thin adhesive layer may have any suitable thickness. For example, a thin adhesive layer may have a thickness from 1 nm to 40 nm, or 5 nm to 20 nm. In some embodiments, when a thin adhesive layer is tens or hundreds of nanometers from the distal opening of the reaction well, the thin adhesive layer has negligible or no effect on the fluorescence yield of fluorescently labeled mononucleotides diffusing through the excitation zone. In some embodiments, when a thin adhesive layer is near (e.g., within 10 or 20 or 30 nm of) the edge of distal opening of the reaction well, the thickness and composition of the thin adhesive layer may be selected to provide optimal enhancement of fluorescence excitation in the excitation zone. In some embodiments, the solid state substrate comprises a plurality of thin adhesive layers. For example, the solid state substrate may comprise a first thin adhesive layer between a first opaque metal layer and a second opaque metal layer, and a second thin adhesive layer between the second opaque metal layer and a dielectric layer such as SiO₂ or SiN.

In some embodiments, the solid state substrate comprises a multilayer structure having a plurality of layers for various purposes. Exemplary multilayer structure include a substrate having the following layers from cis side to trans side listed left to right:
(1) SiN (30 nm), Cr (5 nm), Au (300 nm)
(2) SiN (30 nm), Al (200 nm), Cr (5 nm), Au (300 nm)
(3) SiN (30 nm), SiO₂ (200 nm), Cr (5 nm), Au (300 nm)
(4) SiN (30 nm), SiO₂ (200 nm), Al (200 nm), Cr (5 nm), Au (300 nm)
(5) SiN (30 nm), Al (400 nm)

Throughholes may be fabricated in solid state membranes in a variety of materials including but not limited to, silicon nitride, silicon dioxide (SiO₂), and the like. Although silicon nitride is often symbolized as Si₃N₄ (indicating a Si:N stoichiometry of 3:4), silicon/nitrogen mixtures having other stoichiometric ratios of silicon and nitride may be used. For example, a Si:N stoichiometry close to 3:4 but between 3:4 and 4:4 may have lower structural stress than Si₃N₄.

In general, the methods and substrates of the present invention do not comprise or require protein nanopores or lipid bilayers, thereby avoiding their complexity and instability problems.

Solid state throughholes may be prepared in a variety of ways, as exemplified in the references cited above. In some embodiments a helium ion microscope may be used to drill synthetic throughholes in a variety of materials, e.g. as disclosed by Yang et al, Nanotechnology, 22: 285310 (2011). A chip that supports one or more regions of a thin-film material, e.g. silicon nitride, that has been processed to be a free-standing membrane is introduced to the helium ion microscope (HIM) chamber. HIM motor controls are used to bring a free-standing membrane into the path of the ion beam while the microscope is set for low magnification. Beam parameters including focus and stigmation are adjusted at a region that is adjacent to the free-standing membrane, but on the solid substrate. Once the parameters have been properly fixed, the chip position is moved such that the free-standing membrane region is centered on the ion beam scan region and the beam is blanked. The HIM field of view is set to a dimension (in microns) that is sufficient to contain the entire anticipated reaction well pattern and sufficient to be useful in optical readout (i.e. dependent on optical magnification, camera resolution, etc.). Optionally, the ion beam is then rastered once through the entire field of view at a pixel dwell time that results in a total ion dose sufficient to remove all or most of the membrane autofluorescence, if any (e.g., see WO 2014/066905). The field of view is then set to the proper value (smaller than that used above) to perform lithographically-defined milling of either a single throughhole or an array of throughholes that aligned with the corresponding reaction wells. For example, the throughholes may be made to be coaxial, or not coaxial, with the corresponding reaction wells. The pixel dwell time of the pattern is set to result in throughholes of one or more predetermined diameters, which are optionally determined through the use of a calibration sample prior to sample processing. This entire process is repeated for each desired region on a single chip and/or for each chip introduced into the HIM chamber. Additional guidance for preparing a substrate that comprises reaction wells comprising throughholes in accordance with the invention is provided in Example 1 below.

The depth and diameter of a reaction well, together with the type(s) and thickness(s) of the one or more opaque metal layers of the substrate, may also be selected to achieve an acceptable level of, or to minimize, excitation light in the well away from the distal opening and towards the proximal throughhole. Generally, the intensity of light incident on the distal opening of a well becomes exponentially weaker as it progresses more deeply in to the well towards the throughhole, so that most of the reaction volume is substantially dark, especially near the proximal throughhole. This is particularly so when an opaque metal layer surrounds the distal opening of the reaction well. A benefit of this phenomenon is that a substantial portion or all of the fluorescently labeled polynucleotide strand in the reaction well is protected from unwanted excitation by incident light. This reduces background fluorescence and unwanted modification or degradation of the fluorescently labeled strand. Thus, a deeper well provides greater protection from the incident light, so that a greater proportion of the reaction well is substantially light-free than for a reaction well having the same diameter but smaller depth. A deeper well also provides more space for a longer fluorescently labeled strand to be sequenced. Similarly, a well with a smaller distal opening provides greater darkness in the well away from the distal opening than is provided with a larger distal opening. These general trends are illustrated for example in Table 2 below, which shows light intensities measured in a simulation using Lumerical (March 2020) software available from Lumerical Inc., Vancouver, Canada. More specifically, Intensity was calculated for a well depth 50 nm from the throughhole of the wells (having aluminum side walls) as a fraction of incident light intensity (640 nm) at the distal openings.

**Table 2**

| **Well Diameter (nm)** | **Well Depth (nm)** | **Intensity** |
|---|---|---|
| 50 | 150 | 4 x 10⁻⁴ |
| 100 | 150 | 1.5 x 10⁻² |
| 50 | 250 | 5 x 10⁻⁸ |
| 100 | 250 | 1 x 10⁻⁴ |

In some embodiments, each reaction well has a combination of depth and minimum diameter exemplified by the combinations in Table 3 below.

**Table 3**

| **Depth (nm)** | **Minimum Diameter (nm)** |
|---|---|
| 150-1000 | 30-150 |
| 150-800 | 30-120 |
| 600-1000 | 30-120 |
| 200-1000 | 50-150 |
| 200-800 | 50-150 |
| 200-1000 | 80-150 |
| 200-800 | 80-150 |
| 400-1000 | 80-150 |
| 200-800 | 80-120 |
| 400-1000 | 80-120 |
| 600-1000 | 80-120 |

The substrate, especially surfaces of the substrate that will be in contact with reaction components may also be coated with one or more coatings to impart desirable properties, such as inertness, non-reactivity, or non-affinity towards buffer or reaction components such as exonuclease, fluorescently labeled polynucleotide strands, released mononucleotides, and/or other reaction components or buffer components.

In some embodiments, one or more coatings may be applied to the surfaces of the substrate (also referred to herein as "inner surfaces" of the substrate) that may contact one or more buffer components and/or reaction components that are used or are generated in methods of the present invention. For example, such coatings may help passivate surfaces of the reaction wells to reduce their affinity towards exonucleases and/or mononucleotides. Such coatings may also be used to protect metal components from oxidation or other degradative processes, or to reduce electroosmotic flow (EOF) of buffer ions along such surfaces that can create aqueous flow along the walls of the well.

In some embodiments, the inner surfaces of the reaction well, the inner surfaces of the throughhole, or the inner surfaces of both the reaction well and throughhole comprise at least one coating. In some embodiments, a single coating is applied. In some embodiments, a plurality of coatings is applied. In some embodiments, when a plurality of coatings is applied, the coatings are the same. In some embodiments, when a plurality of coatings is applied, the coatings are not the same. Such coatings may have any suitable thickness selected by the user. For example, a coating may have a thickness from 1 nm to 20 nm, or from 1 nm and 10 nm, or from 2 nm to 10 nm, or from 5 nm to 10 nm. Preferably, the coating thickness is substantially uniform, as may be provided by a variety of methods, such as atomic layer deposition (ALD).

In some embodiments, a coating comprises an inorganic coating. In some embodiments, an inorganic coating comprises a film comprising HfO₂, Al₂O₃, SiO₂, TiO₂, SiN, or Pt. Such a coating may be made by any suitable method. For example, such a coating may be added by ALD. Such coatings are particularly suitable for coating a variety of metal surfaces, such as aluminum, copper, and gold, and also for coating a variety of other types of material surfaces, such as silicon and silicon nitride. In some embodiments, in which the substrate comprises a gold layer, the gold surface may be coated with an organic thiolate compound. See for example Li et al., Bioconjugate Chem. 24(11):1790-1797 (2013). In some embodiments, in which the substrate comprises a non-gold metal or metal oxide layer, the metal or metal oxide surface may be coated with a phosphonic acid-containing compound such as taught by Mutin et al., Chemical Materials 16:5670-5675 (2004), Gao et al., Langmuir 12:6429-6435 (1996), and Zoulalian et al., J. Physical Chemistry B 110:25603-25605 (2006).

In some embodiments, the substrate comprises a dynamic coating comprising polyvinyl pyrrolidone, which may be present in the buffer in which the sequencing methods of the present invention is performed. Such a coating may be particularly suitable for coating SiN, SiO₂, and metal oxides, for example. In some embodiments, such a coating may reduce non-specific binding of exonuclease, mononucleotides, or other buffer or reaction components.

In some embodiments, the solid state substrate comprises a plurality of reaction wells as described in this disclosure, each of which may contain a fluorescently labeled polynucleotide strand for sequencing. The plurality of reaction wells may be arranged in any configuration, such as a random or non-random configuration, and are usually disposed in a plane. In some embodiments, the reaction wells are configured as an array to facilitate the performance of a plurality of sequencing reactions in parallel . In some embodiments, the array comprises a plurality of reaction wells arranged in a linear array. In some embodiments, the array comprises a plurality of reaction wells arranged in a 2-dimensional array of rows and columns. In some embodiments, reaction wells are spaced regularly, e.g., in a rectilinear pattern in which parallel rows are perpendicular to parallel columns (i.e., analogous to x and y axes that are 90 degrees apart). In some embodiments, the rows are not perpendicular to the columns. For example, the rows may be parallel to each other, but the columns may extend at a non-90 degree angle, such as 45 or 60 degrees relative to the rows. In some embodiments, the reaction wells may be configured as a hexagonal array in which columns of wells extend from the rows at a 60 degree angle relative to the directions of the rows. In some embodiments, adjacent wells in each row are separated by the same distance from each other. In some embodiments, adjacent wells in each column are separated by the same distance from each other. In some embodiments, the spacing between adjacent wells in each row and column are separated by the same distance from each other. In some embodiments, the spacing between adjacent rows are different from the spacing between adjacent columns.

When the solid state substrate comprises a plurality of reaction wells, each well is preferably separated from all other wells by a distance that permits fluorescent signals to be unambiguously detected from each well, without substantial interference from fluorescent signals from any other wells. Usually, the minimum distance between adjacent wells depends on (1) the longest wavelength of fluorescent light being detected, and (2) the pixel resolution of the signal detector.

Optical resolution of light signals from two adjacent light sources is often considered to be achieved when the light sources are separated by a distance that is at least one half of the wavelength (λ/2) of the detected light, even if the dimensions of light source are smaller than λ/2 (e.g., when a reaction well has a diameter of 150 nm, 100 nm, or 80 nm). However, greater spacing may be preferred for better resolution to minimize cross-talk interference from light signals from adjacent wells. Thus, for fluorescent signals having a wavelength of 700 nm emanating from adjacent well, a minimum inter-well distance of 350 nm (λ/2) might be sufficient to provide adequate resolution of the two signals. However, a larger inter-well distance would likely improve signal resolution and detection accuracy.

The signal detector may have any suitable pixel resolution that is deemed appropriate by the user. For example, if each pixel of a signal detector has an area of 100 nm x 100 nm, and each reaction well has a diameter of 100 nm, then the light signals from each well are usually collected using a plurality of detector pixels for each well (e.g., a 3 x 3 pixel area, or 4 x 4 pixel area, or 5 x 5 pixel area, per well), to capture most or all of the photons emitted from each well. Generally, using a larger number of pixels for signal detection from a well will provide higher a photon yield (i.e., higher signal intensity) of fluorescent signals collected from each well, provided that the pixel area is not too close to the next adjacent well.

In some embodiments, reaction wells are separated by at least 1 micrometer, or by at least 1.3 micrometers, or by at least 1.5 micrometers, or by at least 1.7 micrometers, or by at least 2 micrometers. However, substrates having reaction well separation distances that are larger or smaller than these inter-well separation distances may also be used.

In some embodiments, the plurality of wells comprising an array of at least 10 times 10 reaction wells, or at least 30 times 30 reaction wells, or at least 100 times 100 reaction wells, or at least 500 times 500 reaction wells, or at least 1000 times 1000 reaction wells.

Exemplary procedures for preparing exemplary solid state substrates are also found in Example 2 below.

As noted above, reacting a fluorescently labeled polynucleotide strand with an exonuclease in a reaction well releases mononucleotides, which are fluorescently labeled mononucleotides or include fluorescently labeled mononucleotides, from the distal end of the strand. During the exonuclease reaction with the fluorescently labeled strand, the trans side of the substrate is illuminated with excitation light to create a fluorescence excitation zone adjacent to the distal opening of the reaction well, so that fluorescently labeled mononucleotides that diffuse through the excitation zone emit fluorescent signals that are detected as a function of time. Stated in a different way, the trans side of the substrate is illuminated with excitation light to create a fluorescence excitation zone adjacent to the distal opening of the reaction well. While the substrate is illuminated, the fluorescently labeled polynucleotide strand is reacted with an exonuclease so that mononucleotides are released serially from the distal end of the strand and diffuse through the fluorescence excitation zone, so that fluorescently labeled mononucleotides in the excitation zone emit fluorescent signals.

The fluorescent signals are detected as a function of time, whereby a nucleotide sequence is determined from the time order of fluorescent signals detected from the released fluorescently labeled mononucleotides.

The production and detection of fluorescent signals may be accomplished using any suitable detector. The detector comprises an excitation source that emits light to illuminate one or more sequencing reaction wells at the same time or at different times.

Typically, the excitation light comprises light that is monochromatic, i.e., the light comprises a narrow wavelength range. If the excitation source emits light that is not monochromatic, the light may be passed through one or more filters to block undesired wavelengths from impinging on the reaction wells. Exemplary light sources include lasers (e.g., argon lasers), light emitting diodes, laser diodes, and lamps, such as xenon and mercury lamps. In some embodiments, the detector comprises one or more free space lasers. In some embodiments, the detector comprises one or more fibers coupled lasers.

In some embodiments, the detector comprises a plurality of light sources, such as two or more lasers or light emitting diodes, each having a selected emission wavelength or emission wavelength range suitable for producing excitation light for exciting selected fluorescent labels of fluorescently labeled mononucleotides that diffuse through the excitation zone of each reaction well.

In some embodiments, the excitation light is circularly polarized. In some embodiments, the excitation light is linearly polarized. In some embodiments, the excitation light is non-polarized. In some embodiments, the excitation light comprises light having a wavelength of 488 nm. In some embodiments, the excitation light comprises light having a wavelength of 532 nm. In some embodiments, the excitation light comprises light having a wavelength of 640 nm. In some embodiments, the excitation light comprises light having a wavelength of 730 nm.

In some embodiments, the excitation light is collimated to illuminate one or more reaction wells, such a plurality of reaction wells that may be configured as an array. In some embodiments, the excitation light is focused, such as in a confocal microscope configuration, which may be used for example for detection of fluorescent signals from a single reaction well.

For embodiments employing multiple fluorescent labels, i.e., when a fluorescently labeled polynucleotide strand comprises different nucleotides comprising different identifying fluorescent labels, the excitation wavelengths may be tailored to balance the relative intensities of emitted light from the different fluorescent labels. Another way to balance the relative intensities of emitted light from the different fluorescent labels is to select the different fluorescent labels based upon the excitation wavelengths produced by the light source or light sources. For example, the intensity of a fluorescent signal emitted by a fluorescent label may be reduced by exciting the label at a wavelength that is shorter than the label's wavelength of maximum absorption (λmax, abs) corresponding to the label's fluorescence emission wavelength. The different fluorescent labels and light source(s) may also be selected to balance and optimize the resolution of emission signals of the different fluorescent labels. This can be accomplished by choosing labels with emission wavelengths that are as far apart from each other as deemed necessary to distinguish signals from the different labels. For example, in some embodiments, labels are chosen with emission wavelength maxima that are at least 20 nm, or at least 25 nm, or at least 30 nm, greater or less than the nearest emission wavelength maxima of the other labels in the fluorescently labeled nucleotides. The choice of labels may also depend on the excitation wavelengths provided by the one or more light sources.

Fluorescence detection may be accomplished using any of a variety of detection modes. Suitable light detectors include, for example, avalanche photodiode detectors; photomultipliers; charge-coupled devices (CCDs), such as intensified CCDs (iCCDs) and electron-multiplying CCDs (emCCDs); complementary metal oxide semiconductor (CMOS) detectors; confocal microscopes; and diode array detectors. Typically, detectors such as CCDs and diode arrays comprise a 2-dimensional array of pixels for collecting fluorescent signals from the reaction wells. As discussed above, the fluorescent signals from a reaction well are usually collected by multiple pixels in the detector, to maximize the collection of photons from that well so that each fluorescent label may be correctly identified. The pixels are usually designed to detect photons over a spectrum of wavelengths that encompass the wavelengths of all of the fluorescent labels that will be released by exonuclease cleavage of the fluorescently labeled strand.

Fluorescence signals are usually monitored continuously for greatest yield of the detected fluorescent signals. Fluorescence signals are detected and/or recorded using a frame rate that is faster than the duration of fluorescent signals emitted by each released mononucleotides passing through the excitation zone. The frame rate may be selected by taking into account fluorescent signal strengths and durations of individual photons or photon bursts emitted by the fluorescently labeled mononucleotides. Fluorescence signals are usually measured after subtraction of background/baseline fluorescence that is measured in the absence of fluorescent mononucleotides.

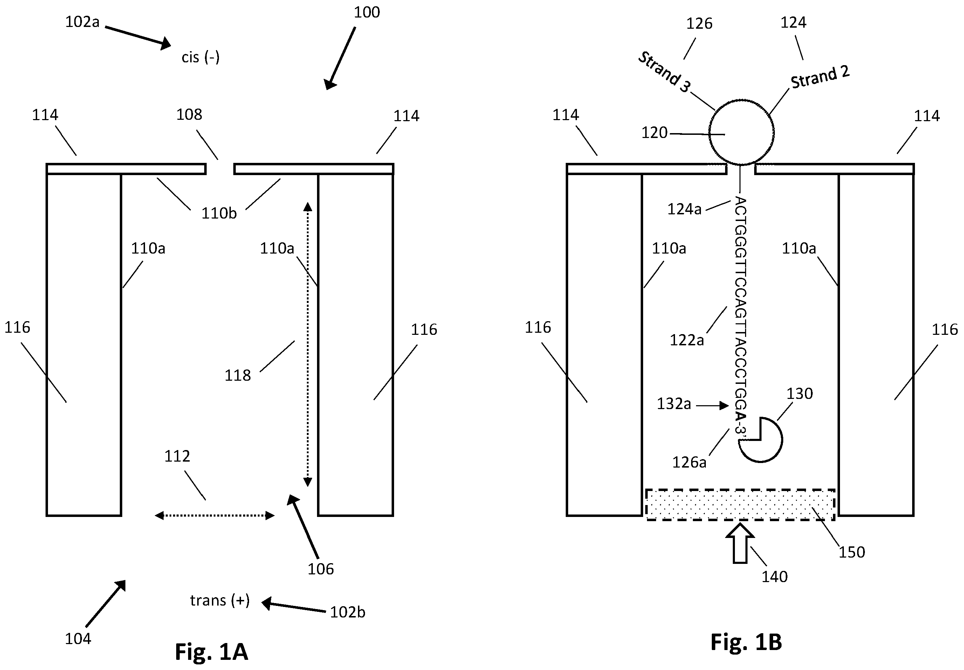

An exemplary solid state substrate comprising a reaction well of the invention is illustrated in the cross-sectional views in Figures 1A to 1D. Substrate 100 comprises a cis side 102a and a trans side 102b. Substrate 100 further comprises a reaction well 104 that defines a reaction volume 106. Reaction well 104 comprises (i) a proximal throughhole 108 extending between the cis side and the trans side of the substrate, (ii) one or more side walls 110a and 110b, and (iii) a distal opening 112. Proximal throughhole 108, which may be cylindrical or non-cylindrical, may be provided as an opening passing through a thin membrane layer 114.

Solid state substrate 100 further comprises an opaque metal layer 116 that substantially blocks excitation light from penetrating into the reaction volume and from penetrating from the trans side to the cis side of the substrate. Although Figures 1A-1D depict a solid state substrate comprising a single opaque metal layer, the solid state substrate may comprise additional layers and materials, as discussed elsewhere herein.

With reference to Figure 1A, reaction well 104 is cylindrical, although reaction wells may have other, non-cylindrical shapes. Reaction volume 106 is defined and enclosed by side walls 110a of layer 116, by side walls 110b of thin membrane layer 114, and by the diameter of the reaction well, which is indicated by a dotted horizontal double-headed arrow spanning distal opening 112. The depth of reaction well 104 is illustrated by a dotted vertical double-headed arrow 118.

The minimum diameter of each reaction well should be large enough to allow at least one exonuclease molecule to diffuse, from the trans side of the substrate, through the distal opening and into the reaction well, and to bind to and digest the distal end of the fluorescently labeled polynucleotide strand. Thus, the minimum diameter of each reaction well is or may be made to be at least as large as the smallest dimension or cross-section of the exonuclease that is used. For example, for an exonuclease having x-y-z dimensions of 6 nm x 6 nm x 6 nm, a minimum diameter of 50 nm or 100 nm or 150 nm are each sufficiently large to provide ample space for an exonuclease to diffuse into a reaction well and serially digest mononucleotides from the distal end of a fluorescently labeled polynucleotide strand.

When an electric field is imposed across the substrate to influence the movement or position of carrier particles loaded with fluorescently labeled polynucleotide strands, cis side 102a is associated with an anodic (negatively charged) electrode, and trans side 102b of substrate 100 is associated with a cathodic (positively charged) electrode.

The cis and trans sides of the solid substrate are contacted with one or more aqueous buffers. In some embodiments, the buffers on the cis and trans sides of the substrate may be the same except for the presence of carrier particles (if present) on the cis side and exonuclease molecules (if present) on the trans side. In other embodiments, the buffers on the cis and trans sides of the substrate are different from each other.

Buffer compositions are provided that are suitable for the sequencing methods of the present invention. Typically, buffers contain buffer molecules, such as HEPES, MOPS, Tris, and phosphate, for example, to maintain a selected pH (e.g., see Sigma-Aldrich Catalog regarding "Good buffers"). Buffer molecule concentrations of 5 mM to 100 mM are typically useful, although higher or lower concentrations can also be used. Salts and other additives, such as NaCl, LiCl, KCl, and glycerol (e.g., 10 mM KCl to 1 M KCl and/or 1-60 or 1-70 volume percent glycerol) and the like can also be included if desired, as well as appropriate cofactors for the particular enzymes that are used (e.g., MgCl₂ or MnCl₂ for some exonucleases). In some embodiments, buffer compositions are constituted to maintain the pH substantially constant at a value in the range of 6.0 to 8.8, although buffers with higher or lower pH values may also be used. For example, for exonuclease III and T4 exonuclease, Buffer 1 or Buffer 2 may be used as follows, in which DTT is dithiothreitol, Ac is acetate, and BSA is bovine serum albumin:
Buffer 1: 10 mM Bis-Tris-Propane-HCl, 10 mM MgCl₂, 1 mM DTT, pH 7.5 at 25°C.
Buffer 2: 50 mM KAc, 20 mM Tris-Ac, 10 mM MgAc₂, 100 µg/mL BSA, pH 7.9 at 25°C.

Another buffer that may be used for T4 exonuclease is Buffer 3:
Buffer 3: 50 mM NaCl, 10 mM Tris-HCl, 10 mM MgCl₂, 100 µg/mL BSA pH 7.9 at 25°C.

To deliver a fluorescently labeled polynucleotide strand to a reaction well, an aqueous solution comprising one or more carrier particles, each comprising one or more attached fluorescently labeled polynucleotide strands, is contacted with the cis side 102a of substrate 100. Each fluorescently labeled polynucleotide strand comprises (i) a proximal end that is attached to the carrier particle, (ii) a distal end that is cleavable by an exonuclease, and (iii) at least one fluorescently labeled nucleotide comprising a fluorescent label. A voltage bias is applied across the substrate using a set of electrodes that establish an electric field from the cis to the trans side of the substrate, typically with one or more anodic electrodes (anodes) on the cis side and one or more cathodic electrodes (cathodes) on the trans side of the substrate. The electric field attracts a carrier particle to the throughhole of a reaction well, so that the distal end of a fluorescently labeled polynucleotide strand on the carrier particle is drawn into and through the proximal throughhole into the reaction volume of a reaction well.

However, the carrier particle does not pass through the throughhole. Since the smallest dimensions of the carrier particles are larger than the smallest diameter of a throughhole, or the carrier particles are otherwise too large to pass through the throughhole, the carrier particles remain on the cis side of the substrate. Furthermore, when the throughholes are dimensioned to allow only one fluorescently labeled polynucleotide strand to enter each reaction well via a throughhole, or if the carrier particle has a sufficiently low loading density of fluorescently labeled polynucleotide strands, only one fluorescently labeled polynucleotide strand is present in the reaction well for digestion by an exonuclease molecule.

The carrier particle and attached fluorescently labeled polynucleotide strand may be held (maintained) in place by maintaining a mild voltage bias across the substrate (between the cis and trans sides) to keep the carrier particle adjacent to, or pressed against, the cis side of the throughhole. The carrier particle is not covalently bonded to the throughhole. The voltage bias is sufficiently mild that it does not cause the fluorescently labeled polynucleotide strand to be released from the carrier particle. The mild voltage bias may be the same as, or different from, the voltage bias that is used to attract the carrier particle to a throughhole to deliver an attached polynucleotide strand to a reaction well.

Figure 1B shows an exemplary reaction well in which a carrier particle 120 has been moved by an electric field (by a voltage bias) to a location adjacent to the cis side of throughhole 108. Carrier particle 120 comprises three fluorescently labeled polynucleotide strands 122a, 122b, and 122c. Each strand is attached to the carrier particle by a proximal end, as illustrated for strand 122a by proximal end 124a, and a distal end that is cleavable by an exonuclease, as illustrated for strand 122a by distal end 126a. Strand 122a has a contiguous nucleotide sequence 5'-ACTGGGTTCCAGTTACCCTGGA-3' (SEQ ID NO.: 1), wherein the 5'-end is attached to carrier particle 120, and the 3'-adenylate (A) is the distal end. For Figures 1B to 1D, all of the nucleotides in strands 122a, 122b, and 122c are fluorescently labeled nucleotides each of which comprises a different fluorescent label that distinguishes each kind of nucleotide (A, C, G and T) from the other kinds of nucleotides.

Although strand 122a in Figure 1B is shown as having only a single-stranded form, it will be appreciated that a fluorescently labeled polynucleotide strand may instead be provided in double-stranded form, or may also comprise one or more single-stranded and double-stranded regions. For example, if fluorescently labeled polynucleotide strand 122a were fully double-stranded, it would be hybridized to a complementary strand comprising the sequence 5'-TCCAGGGTAACTGGAACCCAGT-3' (SEQ ID NO.: 2) as shown below, where base pairing between complementary nucleotides is represented by vertical lines:

5′-ACTGGGTTCCAGTTACCCTGGA-3′
||||||||||||||||||||||
3′-TGACCCAAGGTCAATGGGACCT-5′

As discussed above, if the distal end of a fluorescently labeled polynucleotide strand is double-stranded, then a double-strand-specific exonuclease may be used to cleave the distal end of the fluorescently labeled strand.

With continued reference to Figure 1B, an exonuclease may be introduced by contacting a second aqueous solution that comprises exonuclease molecules with trans side 102b of substrate 100 so that an exonuclease molecule, in this case a 3'-specific single-strand-cleaving exonuclease for cleaving strand 122a. can bind to the distal end of the fluorescently labeled polynucleotide strand in a reaction well, cleave mononucleotides and release them serially from the distal end of the strand. Exonuclease molecule 130 binds distal end 126a of polynucleotide strand 122a and cleaves the phosphodiester linkage (indicated by arrow 132a) between a 3'-terminal A nucleotide and an immediately adjacent G nucleotide.

During reaction of the exonuclease with the fluorescently labeled polynucleotide strand, the trans side of the substrate is illuminated with excitation light 140 to create a fluorescence excitation zone 150 adjacent to the distal opening of the reaction well. It should be noted that fluorescence excitation zone 150, which is illustrated as a shaded region spanning across the diameter of the distal opening and extending both outside and inside the reaction volume of the reaction well, does not have discrete boundaries. Rather, the intensity of incident light in the excitation zone is most concentrated in the vicinity of the distal opening, approximately as shown in Figure 1A, and rapidly diminishes at positions further within or outside of (above) the reaction well (for example, see Table 2 and related discussion above). However, the diameter of the excitation zone is the same, or substantially the same, as the diameter of the distal opening of the reaction well. In other words, the excitation zone does not extend to regions of the solid state substrate beyond the diameter of the distal opening. The 3-dimensional intensity profile of incident light for a reaction well will also depend on the composition(s) and other characteristics of the solid state substrate material(s) around the well (e.g., aluminum, gold, or other material). Thus, as used with reference to the present invention, "adjacent to the distal opening of the reaction well" is intended to refer to the space that is both immediately above and immediately below the plane that passes across the distal opening (and defines the distal end of the reaction volume), such as depicted edge-on by arrow 112 in Figure 1A, and any other nearby illuminated space within the reaction well that causes emission of detectable fluorescent signals by released fluorescently labeled mononucleotides diffusing through that space.

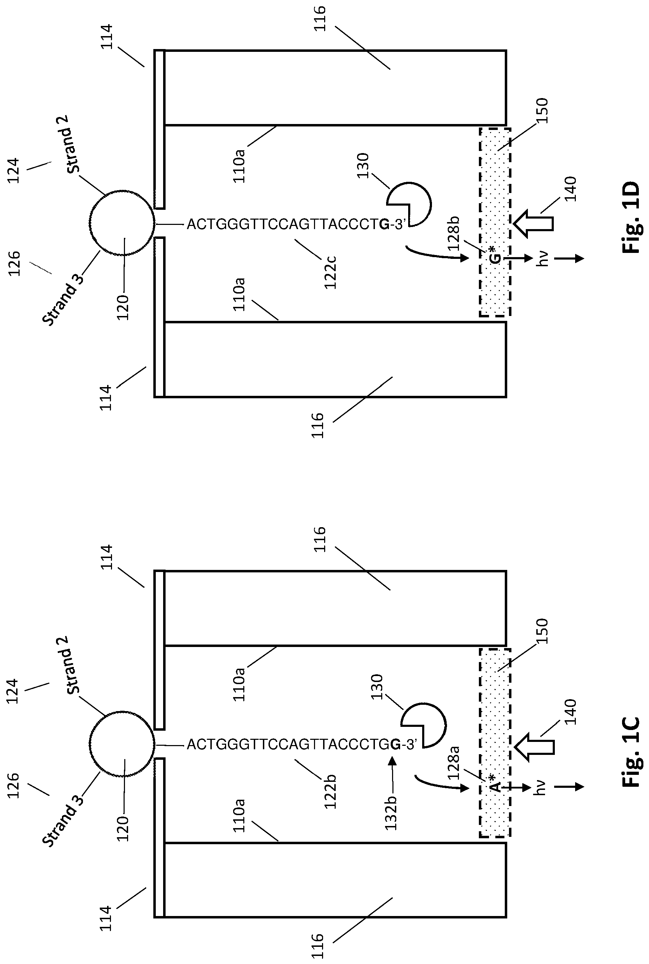

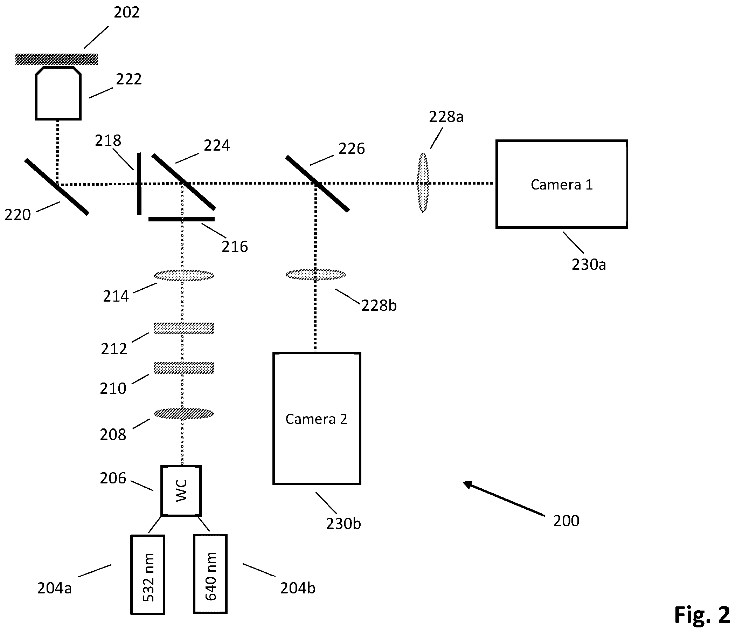

With reference to Figure 1C, a released mononucleotide 128a (shown as A*) diffuses out of the reaction volume and through fluorescence excitation zone 150. While in the excitation zone, fluorescently labeled mononucleotide 128a emits fluorescent signals in the form of multiple (a plurality of) photons (hv) that are detected as an adenylate mononucleotide by a detector (see Figure 2, discussed further below). Notably, passive diffusion provides a sufficient mechanism by which released fluorescently labeled mononucleotides can reach the excitation zone for fluorescence excitation and detection. There is no need for, and the present invention excludes, active bulk flow of aqueous solution into or out of the reaction well during exonuclease cleavage and detection.

Exonucleolytic cleavage and release of the initial 3'-terminal A mononucleotide from strand 122a produces shorter strand 122b having at its distal end a 3'-terminal G. Reaction of the distal end of strand 122b with exonuclease 130 cleaves the phosphodiester linkage (indicated by arrow 132b) between the 3'-terminal G nucleotide and an immediately adjacent G nucleotide, producing shorter strand 122c and a released mononucleotide 128b (shown as G*), as shown in Figure 1D. The released fluorescently labeled mononucleotide 128b diffuses out of the reaction volume and through fluorescence excitation zone 150, emitting fluorescent signals (hv) that are detected by a detector as a guanylate mononucleotide. Exonuclease 130 is then ready to cleave the next 3'-terminal mononucleotide from strand 122c in the same way as for the first two 3'-mononucleotides.

As digestion continues along the fluorescently labeled polynucleotide strand, mononucleotides are released one-by-one, some of which (or all of which, in the case of strands 122abc in Figures 1B-1D) are fluorescently labeled mononucleotides. The released mononucleotides exit the reaction wells by diffusion at a rate that is much greater than the cleavage rate of the exonuclease, so that the released fluorescently labeled mononucleotides serially enter and pass through the excitation zone into bulk solution on the trans side of the reaction well. Unproductive diffusion of mononucleotides through the proximal throughhole is substantially avoided due to blockage of the proximal throughhole by the carrier particle that is attached to the fluorescently labeled polynucleotide strand.

If an exonuclease molecule dissociates from a fluorescently labeled strand before the fluorescently labeled strand has been completely digested, then another exonuclease from solution binds to the distal end of the fluorescently labeled strand and continues digestion. Digestion continues until the fluorescently labeled strand is so short that the exonuclease stops digesting the fluorescently labeled strand or until the cleavage reaction or illumination is otherwise terminated.

Exonuclease-mediated digestion of the fluorescently labeled polynucleotide strands is allowed to proceed for a selected digestion time, or until the yield of reliable fluorescent signals has diminished by a certain amount or below a selected minimum quantity threshold or quality threshold.

After exonuclease-mediated cleavage (also sometimes referred to as "exonuclease-mediated digestion" or "exonuclease digestion") of the one or more fluorescently labeled polynucleotide strands in one or more reaction wells is finished, the reaction wells may be reloaded with new fluorescently labeled polynucleotide strands by applying a reverse voltage bias to the substrate to move the carrier particle away from the throughholes, so that any remaining fluorescently labeled polynucleotide strands are removed from the reaction volumes, and then applying a new voltage bias to (across) the substrate to reload each reaction well with a different fluorescently labeled polynucleotide strands into each the reaction well (i.e., so that the distal end of a different fluorescently labeled polynucleotide strand is delivered into each the reaction well) for reacting with an exonuclease. The new fluorescently labeled polynucleotide strands that are loaded into the reaction wells may be from the same or different carrier particles relative to the carrier particles that provided fluorescently labeled polynucleotide strands in the previous exonuclease cleavage round. Multiple rounds (cycles) of loading fluorescently labeled polynucleotide strands into reaction wells and sequencing the strands using exonuclease-mediated cleavage may be performed until a desired amount of sequence data has been collected or until the sequencing cycles are no longer sufficiently productive.

In some embodiments, reaction wells are loaded with fluorescently labeled polynucleotide strands using a voltage bias of about 250 mV to 500 mV to move the attached carrier particles to the throughholes of the reaction wells, although smaller or larger voltage biases may also be used. In some embodiments, carrier particles are held adjacent to the throughholes using a voltage of about 250 mV to 500 mV, although smaller or larger voltage biases can also be used. In some embodiments, carrier particles are moved away ('ejected") from reaction wells using a voltage bias of about -250 mV to -500 mV, although smaller or larger negative voltage biases may also be used.

One advantage of re-using the carrier particles to deliver multiples fluorescently labeled polynucleotide strands to one or more reaction wells is that a large number of fluorescently labeled polynucleotide strand can be sequenced from a single solid state substrate and single nucleic acid sample. Another advantage is that collecting sequence data from a greater proportion of the total nucleic acid sample population can improve the completeness of the sequence data, reduce gaps, and/or increase the collection of redundant sequence data to align sequences and formulate optimal consensus sequences.

An exemplary sequencing apparatus 200 comprising a solid state substrate of the invention is illustrated in Figure 2. A substrate 202 having a cis side and a trans side is placed adjacent to a microscope objective lens 222, such that the trans side of the substrate faces the lens. Excitation light is delivered to, and fluorescent light signals are received from, the excitation zone of one or more reaction wells in substrate 202. Light paths are illustrated by dotted lines.

The apparatus also comprises excitation light sources 204a and 204b to provide excitation light having selected wavelengths, such as wavelengths of 532 nm and 640 nm, respectively, that are selected to excite fluorescent labels of mononucleotides that are released from a fluorescently labeled polynucleotide strand by exonuclease activity. Here, the apparatus comprises two fiber-coupled laser light sources 204a and 204b. Excitation light beams having different wavelengths from sources 204a and 204b are passed through wavelength combiner (WC) 206 (a fiber based wavelength combiner), fiber optic connector 208, collimating lens 210 (e.g., an achromatic doublet lens, Thorlabs #AC254-060-A), optional shutter 211, and then through quarter wave plate 212 (e.g., an achromatic quarter wave plate, Thorlabs AQWP05M-600), to convert the linearly polarized beam from the lasers into a circularly polarized beam. The light then passes through focusing lens 214 (e.g., an achromatic doublet lens from Thorlabs AC254-400-A), to focus the beam to the back focal plane of the microscope objective lens, and then through multiband excitation filter 216 (e.g., from Chroma Technology ZET 532/640x). After passing through filter 216, the light is reflected off multiband dichroic beamsplitter 218 (e.g., Chroma Technology ZT 532/640rpc) to mirror 220, through microscope objective 222. and onto the trans side of substrate 201. In some embodiments, microscope objective 222 is an oil immersion microscope objective (e.g., Olympus APON60XOTIRF). In some embodiments, objective 222 is a water immersion microscope objective. In some embodiments, objective 222 is an air microscope objective.

Impingement of the excitation light on the trans side of substrate 200, particularly on the distal opening(s) of one or more reaction wells of substrate 200, creates fluorescence excitation zones (see Figures 1B-1D) that are adjacent to the distal openings of the reaction wells.

When fluorescently labeled mononucleotides are released serially from the distal end of a fluorescently labeled polynucleotide strand and diffuse out of the reaction volume through the distal opening of a reaction well, the mononucleotides diffuse through the excitation zone and emit fluorescent light in response to the excitation light. The emitted fluorescent light is collected and focused by microscope objective 222 and is reflected by mirror 220 through multiband dichroic beamsplitter 218, through multiband emission filter 224 (e.g., Chroma Technology ZET 532/640m) to dichroic beamsplitter 226 (e.g., Chroma Technology T6351pxr). In the apparatus of Figure 2, the emitted fluorescent light that passes through dichroic beamsplitter 226 is focuses by lens 228a (e.g., Olympus SWTLU-C tube lens) and then onto detector camera 230a for detection; and emitted fluorescent light that is reflected by dichroic beamsplitter 226 is focuses by lens 228b and then onto detector camera 230b for detection (e.g., Hamamatsu C13440-20CU Orca Flash 4.0). Detector cameras 230a and 230b are preferably synchronized to properly detect the time order of fluorescent signals from the released fluorescently labeled mononucleotides.

For more colors, the two cameras may be replaced with a single camera, and a prism may be used instead of a focusing lens 228a, by which emitted light signals are angularly separated by wavelength onto different regions of the detector field for individual quantification and identification of nucleotide-specific fluorescent signals. Alternatively, a third and fourth camera may be included with attendant lenses and beam splitters to capture more than two different fluorescent signals.

Sequencing apparatus for use in the present invention may also comprise a computer and software for collecting and processing fluorescent signal data.

Since the portion of the fluorescently labeled polynucleotide strand that is in the reaction volume between the reaction well throughhole and the excitation zone is not substantially illuminated, the non-illuminated fluorescent nucleotides in that portion of the fluorescently labeled strand do not emit fluorescent light signals, or if they do, such signals are negligible. Only fluorescent mononucleotides that are excited while in the excitation zone emit a fluorescent signal that is detected by the detector. Also, if the distal opening is defined by a side wall comprising gold, then excitation intensity of the incident light may be greater than for aluminum, providing an enhancement of fluorescence, in other words, a greater flux of fluorescent photons for detection.

The excitation light that impinges on the distal openings of each reaction well may be oriented so as to be orthogonal to the substrate surface, in other words, parallel to the central axis of each reaction well, e.g., when the reaction wells are cylindrical in shape. Alternatively, the excitation light that impinges on the distal opening of each reaction well may be oriented so as to be non-orthogonal to the substrate surface.

The wavelength of the excitation light is also selected to be compatible with the fluorescent labels of the released mononucleotides, so that when the excitation light impinges on each fluorescent label, the fluorescent label absorbs the excitation light and then emits photons having a wavelength that is longer (lower energy) than the wavelength of the excitation light. The difference between the wavelength of maximum absorption and the wavelength of maximum emission associated with a fluorescent label is known as the Stokes shift.

Fluorescence signals are detected using a detector frame rate that is faster than the shortest time windows during which fluorescently labeled mononucleotides emit individual photons or photon bursts while they diffuse through the excitation zone. Usually, released fluorescently labeled mononucleotides enter the excitation zone within 10, or within 20, or within 50 milliseconds, whereas they are released one-by-one by exonuclease-mediated cleavage at time intervals between about 100 milliseconds and about 10 seconds. Usually, released fluorescently labeled mononucleotides enter the excitation zone within 10 milliseconds after being cleaved from a fluorescently labeled strand. Frame rates may also be selected based in part on the size and speed of memory, signal to noise, and fluorescent signal strength.

Exonuclease cleavage rates and the diffusion times (or diffusion speeds) of released fluorescently labeled mononucleotides may be adjusted by varying reaction parameters such as pH, viscosity, temperature, and choice of exonuclease. For example, viscosity may be increased by including a viscous additive such as glycerol, e.g., at a concentration of from 1% to 60% (v:v), or from 1% to 70% (v:v), or from 50% to 70% (v:v), in an aqueous buffer on the trans side of the substrate. In some embodiments, an aqueous buffer on the trans side of the substrate comprises about 50% to about 60% glycerol (v:v), or about 50% to 70% (v:v), or about 50% glycerol (v:v), or about 60% glycerol (v:v), or about 70% glycerol (v:v). The presence of an increased viscosity in and around the detection zone can help reduce mononucleotide diffusion speeds (and provide longer mononucleotide dwell times) during fluorescence detection, providing several benefits, such as (1) higher fluorescence signals due to the collection of more emitted photons for each fluorescent mononucleotide passing through the detection zone, (2) higher signal to noise, (3) the ability to use lower laser power if desired, thereby generating less heat, (4) less cross-contamination (if any) from fluorescent mononucleotides diffusing from a reaction well towards an adjacent reaction well, (5) and the ability to use place reaction wells more closely together in an array.

Each released fluorescently labeled mononucleotide may be identified (e.g., as A, C, G or T) from the characteristics of the measured fluorescent signal, such as (1) the particular emission wavelength or peak shape of fluorescence of the fluorescent label associated with each different kind of nucleotide, (2) signal intensity, which may be measured as a sum of multiple photons from the same mononucleotide during transit through the excitation zone of a reaction well, and (3) the absence of contributions of fluorescence signals from any other released mononucleotide. For example, fluorescently labeled mononucleotides that diffuse out of the excitation zone of a first reaction well into the excitation zone of a second reaction zone can be excluded from the fluorescent signals detected for the second well based on the trajectory of movement of the fluorescently labeled mononucleotide towards the second well. Similarly, fluorescently labeled mononucleotides that diffuse out of, and then return to, the excitation zone of a first reaction well can be excluded from the fluorescent signals detected for the first well based on the trajectory of movement of the fluorescently labeled mononucleotide returning towards the first well.

Also, the fluorescently labeled mononucleotides that are used in the present invention may be selected to be moderately susceptible to photobleaching under the illumination conditions of the substrate, so that fluorescently labeled mononucleotides that diffuse out of the excitation zone are substantially bleached, and thus rendered non-fluorescent, by the incident excitation light after the mononucleotide label has been detected in the excitation zone of the first reaction well, before it returns to the same excitation zone or diffuses to another excitation zone. Fluorescence signals from inactive reaction wells can be disregarded by computer software.

Sequences may be assembled from sequence data obtained from multiple polynucleotide fragments that contain the same or overlapping sequence regions. In some embodiments, determination of a polynucleotide sequence includes determining sequences of subsets of the full set of four natural nucleotides, A, C, G and T for DNA (or A, C, G or U for RNA), such as, for example, a sequence of only A's and C's of a target polynucleotide. In some embodiments, determination of a polynucleotide sequence includes the determination of the identities, order, and locations of one, two, three or all of the four types of nucleotides within a target polynucleotide. In some embodiments, determination of a polynucleotide sequence includes the determination of the identities, order, and locations of two, three or all of the four types of nucleotides within a target polynucleotide. In some embodiments, determination of a polynucleotide sequence includes determining one or more subsequences of a target polynucleotide that serve as a fingerprint for the target polynucleotide, such as subsequences that uniquely identify a target polynucleotide, or a class of target polynucleotides, within a set of polynucleotides, e.g. all different RNA sequences expressed by a cell.

The present disclosure also provides kits that may be useful in performing methods of the invention. Generally, a kit may be any delivery system for delivering materials or reagents for carrying out a method of the invention. In the context of reaction assays, such delivery systems include systems that allow for the storage, transport, or delivery of reaction reagents (e.g., nucleotide 5'-triphosphates comprising fluorescent labels, such as mutually quenching fluorescent labels, enzymes, carrier particles, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. Such contents may be delivered to the intended recipient together or separately. For example, a first container may contain an enzyme for use in an assay, while a second or more containers contain mutually quenching fluorescent labels. In some embodiments, a kit may include one or more of the following: an exonuclease, a template-dependent DNA polymerase or RNA polymerase, one or more fluorescently labeled nucleotide 5'-triphosphates, buffers, and carrier particles that comprise affinity labels such as avidin, streptavidin, polynucleotides that are complementary to polynucleotides that are to be sequenced, etc.

### Example 1

### Conjugation of Nucleic Acids to Carrier Particles

A. Selection and Design of DNA for Attachment to Carrier Particles. For direct conjugation of DNA to gold or silver nanoparticles or to gold or silver surfaces, single or multiple (2 - 6) thiol groups are attached on either the 3' or 5' end of the oligonucleotide (5'-GCTATGTGGCGCGGTATTAT-3') (SEQ ID NO.: 3). Single thiols are obtained from commercially available precursors, 3'- or 5'- disulfide (CH₂)ₙ-S-S-(CH₂)ₙ-OH (n = 3 or 6) modified oligonucleotides (IDT, Iowa). Two thiol groups are introduced via conjugation of commercially available 3'- or 5'- amino-functionalized oligonucleotide (IDT, Iowa or TriLink) with (±)-α-lipoic acid. Alternatively, multiple thiols are introduced with 1, 2 or 3 consecutive DTPA phosphoramidites on either 3'- or 5'-end of oligonucleotide (IDT, Iowa). For conjugating DNA to functionalized nanoparticles, an appropriate complementary conjugation groups (amine, thiol, DBCO, BCN) is attached to either the 3' or 5' end of the oligonucleotide. Linkers of various lengths are introduced between the conjugation moiety and the oligonucleotide in order to facilitate access of enzymes (e.g., access to a polymerase if a fluorescently labeled polynucleotide strand complementary to a sample nucleic acid strand will be synthesized by DNA or RNA polymerase using a template that is immobilized on the carrier particle) oligonucleotides to the oligonucleotide sequence. Examples of linkers are thymidine monophosphate × n (n = 1 - 40), PEG3, PEG4, PEG5, and (PEG6-P(O)(OH)O-) × n (n = 1 - 12), where PEG means polyethylene glycol, and PEGN for N = 3, 4, 5 and 6 means a polymer of N ethylene glycol units.

B. Conjugation of DNA to Gold Nanoparticles. Oligonucleotides functionalized at 3'- or 5'-end with acyclic disulfide group (CH₂)ₙ-S-S-(CH₂)ₙ-OH (n = 3 or 6), 1, 2 or 3 DTPA (from IDT, Iowa) or (±)-α-lipoic acid are incubated with a 50-fold excess of a reducing agent (tris(2-carboxyethyl)phosphine (TCEP) or dithiothreitol (DTT) in phosphate buffer at pH 7.4 for 45 min. The reduced thiol-functionalized oligonucleotides are purified by desalting with gel filtration columns packed with Sephadex G-25 and milli-Q water as eluent.

Purified oligonucleotides are mixed with gold nanoparticles (10 nm, 15 nm, 50 nm, and 100 nm diameter from Sigma-Aldrich) at various molar ratios (100:1, 300:1, 500:1, 1000:1, 3000:1, 10000:1, 20000:1, etc.) and the pH is adjusted to 4.3 with 50 mM citrate-HCl buffer, or to pH 7.4 or pH 8 with 10 mM sodium phosphate buffer. Tween 20 is added to a final concentration of 0.02%, 0.05% or 0.1%, or instead of Tween 20, SDS is added to a final concentration of 0.01%, 0.025%, or 0.05%. After initial incubation at ambient temperature for 1 to 24 hours, 4 M NaCl is added in small portions up to 1 M total concentration. The reaction mixture is further incubated for 1 to 24 hours. Optionally, to block unreacted surface areas on the gold nanoparticles (also referred to as "back-filling"), a water-soluble oligo(ethylene glycol)-alkylthiol, where oligo(ethylene glycol) is PEG3 or PEG6, and alkyl is (CH₂)₈ - (CH₂)₁₂, is added to the reaction mixture (variable ratios relative to thiol-modified oligonucleotide: 1:1, 4:1, 10:1, 25:1, 100:1) and the reaction mixture is incubated for 15 min to 24 hours. Oligonucleotide-nanoparticle conjugates are harvested by centrifuging and multiple washing with appropriate buffer (e.g. 10 mM phosphate buffer pH 8 with 0.02% Tween 20). Nanoparticles are stored at 4 °C in the same washing buffer before further use.

For quantitation of DNA loading, oligonucleotides are released from nanoparticles via etching with KCN / K₃[Fe(CN)₆] mixture or DTT. Released oligonucleotides are quantified with a fluorometer using SYBR^{™} Gold or OliGreen^{™} (ThermoFisher Scientific) as a staining agent. For internally labeled oligonucleotides that are obtained by primer extension in presence of at least one fluorescently labeled (Cy3, Cy5, Cy7, Alexa Fluor 488 etc.) deoxynucleotide triphosphate (dATP, dCTP, dGTP or dUTP), intrinsic absorbance or fluorescence is used as a readout for quantification after nucleotide release from nanoparticles. Alternatively, reversible hybridization of suitably labeled complementary oligonucleotide is used for quantification.

Table 4 shows DNA loading data obtained with carrier particles comprising different quantities of an oligonucleotide 5'- GCTATGTGGCGCGGTATTAT-3' (SEQ ID NO.: 3, supra) as a capture moiety and/or primer conjugated to gold nanoparticles for use in subsequent primer extension with fluorescent dNTPs or for annealing with primer extension products prepared separately.

**Table 4***

| Au Diam. (nm) | No. of 3' or 5' Thiols | Linker | Au Particle Vendor | Conjugation Conditions | Back-Filling | Measured DNA:Au Loading |
|---|---|---|---|---|---|---|
| 15 | 1 | PEG6x12 | SA | (1) | none | 215 |
| 15 | 2 | T40 | SA | (1) | none | 288 |
| 15 | 1 | PEG6x12 | SA | Citrate pH 4.3 | none | 64 |
| 15 | 1 | PEG6x12 | SA | citrate | C11PEG6OH | 4-41 |
| 15 | 1 | PEG6x12 | SA | citrate | none | 195 |
| 15 | 1 | PEG6x12 | TP | Salting-aging | none | 227 |
| 15 | 1 | PEG6 | TP | Salting-aging | none | 269 |
| 15 | 1 | PEG6x12 | C | citrate | none | 44 |
| 15 | 1 | PEG6x6 | C | citrate | none | 59 |
| 15 | 1 | PEG6x6 | SA | citrate | none | 152 |
| 15 | 1 | PEG3 | SA | citrate | none | 92-159 |
| 50 | 1 | PEG6x12 | SA | citrate | none | 503-637 |
| 50 | 1 | PEG6x12 | SA | Salting-aging | none | 950 |
| 50 | 1 | PEG6x12 | SA | citrate | C11PEG6OH | 786 |
| 50 | 2 | PEG6 | SA | Salting-aging | none | 1050 |
| 50 | 2 | PEG6 | SA | Salting-aging | C11PEG6OH | 990 |
| 50 | 1 | PEG6x6 | SA | Salting-aging | none | 902 |
| 50 | 1 | PEG6x3 | SA | Salting-aging | none | 975 |
| 50 | 1 | T20 | SA | Salting-aging | none | 531 |

| | | | | | | |
|---|---|---|---|---|---|---|
| *T₄₀ is a 40-mer of thymidines; Au particle vendors were Sigma Aldrich (SA), Ted Pella (TP), and Cytodiagnostics (C); conjugation conditions were (1) phosphate pH 7.4 followed by 1M NaCl overnight (salt aging), or (2) 50 mM citrate-HCl pH 4.3. | | | | | | |

C. Amino-Functionalized DNA; Amide Coupling. Carboxylic acid-functionalized nanoparticles (commercially available quantum dots, polymer-coated gold or iron oxide nanoparticles - e.g., P/N 900226, 747254, 765481, 900475 from Sigma-Aldrich, P/N Q21301MP from ThermoFisher Scientific, P/N MP25-CA from Nanocs or P/N SC0050 from Ocean Nanotech) are suspended in sodium borate buffer (pH 8-9) and treated with N-(3-dimethylaminopropyl)-N'-ethylcarbodiimide hydrochloride (EDC·HCl; 100 - 2000 eq) and N-hydroxysulfosuccinimide (sulfo-NHS, 100-2000 eq) for 1 to 2 hours at ambient temperature. Then amino-functionalized DNA (1 eq) is added to the mixture. Optionally, 4M NaCl is added in small portions up to 0.15 M - 0.5 M (depending on the colloidal stability of particular nanoparticles). The reaction mixture is incubated for 3 to 24 hours. DNA-nanoparticle conjugates are purified by centrifuging and subsequent washing or by size-exclusion chromatography followed by dialysis.

D. DBCO or BCN-Functionalized DNA; Copper-Free Click Reaction Coupling. Carboxylic acid-functionalized nanoparticles (commercially available quantum dots, polymer-coated gold or iron oxide nanoparticles - e.g., P/N 900226, 747254, 765481, 900475 from Sigma-Aldrich; P/N Q21301MP from ThermoFisher Scientific; P/N MP25-CA from Nanocs or P/N SC0050 from Ocean Nanotech) are coupled with an appropriate azido-amine per the protocol above. Azido-functionalized nanoparticles (prepared above) are mixed with 3'- or 5'-DBCO (dibenzocyclooctyne)- or BCN (bicyclooctyne)-functionalized DNA (commercially available from IDT or prepared from 3'- or 5'- amino-functionalized DNA via coupling with corresponding NHS ester, e.g. P/N A102 from Click Chemistry Tools or P/N BP-22851 from BroadPharm) in appropriate reaction buffer (10 mM - 50 mM sodium phosphate, pH 7 - pH 8). Optionally, 4 M NaCl is added in small portions up to 0.15 M - 0.5 M (depending on the colloidal stability of particular nanoparticles). The reaction mixture is incubated for 3 to 24 hours. DNA-nanoparticle conjugates are purified by centrifuging and subsequent washing or by size-exclusion chromatography followed by dialysis.

E. Thiol Functionalized DNA; Michael Addition to Maleimide. Amine-functionalized nanoparticles (commercially available quantum dots, polymer-coated gold or iron oxide nanoparticles - e.g., P/N 900290, 765341, 747327 from Sigma Aldrich) are coupled with succinimidyl 4-(N-maleimidomethyl) cyclohexane-1-carboxylate (SMCC) at pH 7.0 - pH 7.4 following an established protocol (e.g. Thermo Fisher). Maleimide-functionalized nanoparticles (commercial, e.g. 900461 from Sigma Aldrich, or prepared above) are mixed with thiol-functionalized DNA (deprotected with TCEP or DTT and purified as described above) in a 10 mM - 50 mM sodium phosphate buffer, pH 7.0 - pH 7.4). Optionally, 4 M NaCl is added in small portions up to 0.15 M - 0.5 M (depending on the colloidal stability of particular nanoparticles). The reaction mixture is incubated for 0.5 - 24 hours. DNA-nanoparticle conjugates are purified by centrifuging and subsequent washing or by size-exclusion chromatography followed by dialysis.

### Example 2

### Solid State Substrates

Substrate Preparation. A 300 µm thick 100 mm double-side-polished silicon wafer is prepared having a 30 nm layer of SiN deposited by low pressure chemical vapor deposition (LPCVD) on each side of the wafer (e.g., from Virginia Semiconductor, Fredericksburg, VA, or Rogue Valley, OR). Negative e-beam resist is spun on each side and the resist on one side (the "front" side) of the wafer is then exposed in an e-beam lithography (EBL) instrument to pattern reaction wells over the SiN layer on that side. The resist is then developed, and unexposed resist is removed. A 5 nm adhesion layer of chromium or titanium is deposited by e-beam evaporation onto the front side of the wafer, followed by e-beam deposition of a selected thickness of an opaque metal layer (e.g., 200 nm of Au or Al) onto the front side of the wafer. The wafer is then placed in a solution that removes the remaining exposed resist (a "lift off" step) from the front side, leaving reaction wells in the metal film layer that have diameters of 40 to 120 nm and well depths of 100 to 250, or other dimensions according to the preference of the user.

The back (non-patterned) side of the wafer is then patterned via conventional photolithography with a positive tone resist to expose a square window aligned with the front EBL features. These features are etched with reactive ion etch (RIE) through the SiN layer of the back side of the wafer down to the Si layer. The wafer is then mounted in a holder with an O-ring that protects the metal-coated side from KOH solution and is then immersed in KOH solution, which preferentially etches down the (100) plane until the opposite SiN membrane is reached, resulting in a free-standing SiN + metal substrate with reaction wells on the front side of the wafer that are open down to the SiN layer.

Reaction Well-Aligned Throughhole Fabrication. The above-described substrate is then loaded into a focused ion beam (FIB) instrument (e.g., a Zeiss Orion NanoFab in GFIS mode with helium) to create throughholes at the base of each reaction well. Throughholes are milled in free-standing SiN membranes as described previously (Marshall et al., Direct and transmission milling of suspended silicon nitride membranes with a focused helium ion beam. Scanning 34:101-106 (2012)). Briefly, the ion beam is aligned and the substrate to be milled is brought into focus on the tool. Throughholes are milled in the free-standing SiN layer of the substrate by exposing points or rastering over shapes in the substrate for a given dwell time and beam current relative to the thickness of the substrate, to target a desired final size and shape. Other tools capable of fabricating small throughholes include TEMs and other varieties of FIB (for example, gallium or gas field ion source (GFIS) neon). The throughholes may also be lithographically formed by overlaying a second EBL patterning step (as described in the above substrate preparation section) subsequent to creating the larger reaction well. Overlay of the second feature over the first feature yields a pattern consisting of the second feature (reaction well) concentrically aligned with the smaller first feature (throughhole). The resulting pattern may be used directly or, if the second feature is larger than a desired final size, it can be reduced with sub-nanometer precision by atomic layer deposition (ALD) of films such as HfO₂, Al₂O₃, SiO₂, TiO₂, SiN, or Pt.

### Example 3

### Detection of Exonucleolytically Released Mononucleotides

A. Formation of Fluorescently Labeled Polynucleotide Strand. Double-stranded fluorescently labeled DNA was prepared by template-dependent polymerase-mediated extension of a 5'-bis-biotinylated primer. The 5'-bis-biotinylated primer contained two biotin groups attached to its 5' end to enhance non-covalent binding to streptavidin.

The structure of the bis-biotin moiety was the following, in which the 3'-phosphate is attached to the 5'-hydroxyl of the 5'-terminal nucleotide of the primer:

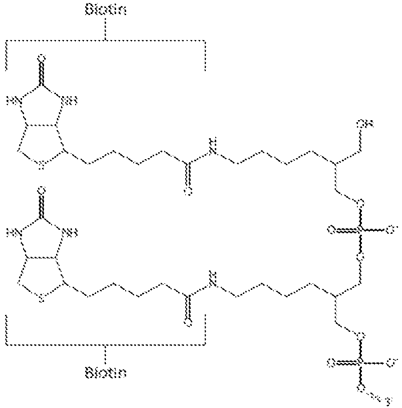

The nucleotide sequence of the primer was:
5'-(bis-biotin)-GCTATGTGGCGCGGTATTAG-3' (SEQ ID NO.: 4).

The sequence of the hepatitis B virus (HBV) template (called "HBV-pos=1980") was:

5'-GACTACTAGGTCTCTAGACGCTGGATCTTCCAAATTAACACCCACCCAGG
TAGCTAGAGTCATTAGTTCCCCCCAGCAAAGAATTGCTTGCCTGAGTGCA
GTATGGTGAGGTGAACAATGCTCAGGAGACTCTAAGGCTTCCCGATACAG
AGCTGAGGCGGTATCTAGAAGATCTCGTACCTAATACCGCGCCACATAGC-3'
(SEQ ID NO.: 5).

The first 180 5'-nucleotides of the template corresponded to nucleotides 1980 to 2159 of the HBV genome. The last twenty 3'-terminal nucleotides (underlined) of the template were complementary to the primer sequence. The last five 3'-terminal nucleotides of the template were synthesized to contain phosphothioate internucleotide linkages to inhibit exonuclease degradation of the 3'-end of the template.

Template-dependent primer extension was performed in the presence of ATP, GTP, Cy3b-dCTP, and Cy5-dUTP under the following conditions: 20 mM Tris-HCl, 10 mM (NH4)2SO4, 10 mM KCl, 4 mM MgSO4, 0.1% Triton^{®}-X-100, pH 8.8 at 25 °C, 1 mM DTT, 1% Formamide (v/v), 10% glycerol (v/v), 0.1 mM dNTPs, 0.8 micromolar DNA template, and 2 micromolar primer. This yielded a double-stranded nucleic acid comprising the HBV template sequence hybridized to the complementary primer extension product, wherein the primer extension product comprised a fluorescently labeled polynucleotide strand that contained fluorescently labeled cytidine and uridine bases.

The predicted sequence of the fluorescently labeled portion of the primer extension product, which corresponded to the complementary sequence of the first 180 5'-nucleotides of SEQ ID NO.: 5 (disregarding the first 5'-terminal 20 nucleotides, which were from the unlabeled primer), was:

5'-GUACGAGAUCUUCUAGAUACCGCCUCAGCUCUGUAUCGGGAAGCCUUAGA
GUCUCCUGAGCAUUGUUCACCUCACCAUACUGCACUCAGGCAAGCAAUUC
UUUGCUGGGGGGAACUAAUGACUCUAGCUACCUGGGUGGGUGUUAAUUUG
GAAGAUCCAGCGUCUAGAGACCUAGUAGUC-3' (SEQ ID NO.: 6).

B. Attachment of Fluorescently Labeled Polynucleotide Strand to Exemplary Carrier Particles. Streptavidin-coated iron oxide nanoparticles having average diameters of 50 nm (part number SV0050 from Ocean Nanotech) were mixed with the biotin-functionalized DNA in a reaction buffer (10 mM PBS pH 7.4, containing 10 mM sodium phosphate, 134 mM sodium chloride) at various molar ratios (10:1, 5:1, 2:1, and 1:1). Tween 20 was added to a final concentration of 0.01% (v/v), and the attachment (conjugation) reaction mixtures were incubated for 4 to 14 hours at 25 °C with shaking at 250 rpm. After the conjugation reactions were complete, the reaction mixtures were centrifuged at 15,000 rpm for 10 min, and the nanoparticle-free, DNA- containing supernatants were removed. DNA-nanoparticle conjugates were further purified by (1) centrifugation, (2) subsequent centrifugal washing with 10 mM PBS buffer pH 7.4 containing 0.05% (v/v) Tween-20, and (3) re-suspension in the same washing buffer. The DNA-conjugated nanoparticles were stored at 4 °C.

C. Loading of Fluorescently Labeled Polynucleotide Strand into Reaction Well. A solid state substrate comprising a cis side and a trans side was prepared using the techniques described above. The solid state substrate comprised a 4 x 4 square array of reaction wells having radially symmetric side walls with an internal diameter of ~40 nm, and a well depth of about 315 nm. The side walls of the reaction wells were formed by ion bombardment of a membrane defined within the substrate, the membrane comprising, from the trans side to the cis side, planar layers of (1) aluminum (250 nm), (2) titanium (15 nm), and (3) SiN (30 nm). In addition, the trans and cis sides of the membrane, and the inner side walls of the reaction well, were coated with a 10 nm thick coating of SiO₂ that was added by atomic layer deposition. In this example, the internal diameters of the proximal throughhole, the reaction volume surrounded by the radially symmetrical side wall, and the distal opening of each reaction well were approximately the same.

The reaction wells were separated from each other by a pitch of 1500 nm in the x and y directions. The solid state substrate was assembled in a sequencing cartridge such that the cis and trans sides of the substrate were fluidically accessible. The solid state substrate in the sequencing cartridge was positioned as shown schematically for substrate 202 in Figure 2, so that the distal openings of the reaction wells on the trans side of the solid state substrate were in the focus of the excitation laser beam that passed through microscope objective 222. The fluidic chamber close to the objective is referred to as trans chamber, and the distal chamber is referred to as the cis chamber. Each chamber was filled with a reaction buffer containing 20 mM Tris-HCl pH 7.5, 10mM MgCl₂, and then 25 pmole of DNA-conjugated carrier particles in the same buffer were added to the cis chamber (final concentration 100 pM nanoparticles with ~7 DNA strands/nanoparticle).

The chambers were electrically connected via two electrodes (Ag/AgCl₂ electrodes). These electrodes were used to apply a cis-to-trans 300 mV voltage bias across the substrate to electrophoretically transport the DNA-conjugated nanoparticles to the nanopore reaction wells, so that negatively charged fluorescently labeled polynucleotide strands (hybridized to the complementary template strand in a dsDNA complex) attached to the carrier particles were electrophoretically drawn through the proximal throughholes and into the reaction volumes of the reaction wells.

Plugging of the proximal throughholes with carrier particles was monitored by measuring the cis-to-trans current from a starting (open, unplugged) current of approximately 1000 nAmp to a current below about 200 nAmp when the rate of change of the current had plateaued. Plugging of all 16 wells was complete in about 30 seconds. Because the diameters of the carrier particles were greater than the diameters of the reaction wells, the carrier particles were prevented from passing through the reaction wells from the cis side of the substrate to the trans side. In addition, because the carrier particles were sparsely loaded with fluorescently labeled polynucleotide strands, no more than one fluorescently labeled polynucleotide strand was loaded into each reaction well.

After the proximal throughholes of the reaction wells were plugged, and fluorescently labeled polynucleotide strands were loaded into the reaction wells, the trans chamber buffer was replaced with the following exonuclease reaction buffer: 20 mM Tris-HCl pH 7.5, 10 mM MgCl₂, 50% to 70% (v/v) glycerol, and 20 nM E. coli exonuclease III.

Immediately after introducing the exonuclease reaction buffer, laser illumination of the trans side of the substrate and camera recording of fluorescence emissions from the distal ends of the reaction wells were started. Camera frame rates for emission signal detection was usually between 200 and 500 frames per second (fps), and laser light intensities at 535 nm and 648 nm were usually from 5 mWatt to 15 mWatt.

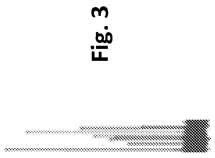

Figure 3 shows an exemplary sequencing profile obtained for the first nine nucleotides from exonucleolytic cleavage of the 3'-end of a fluorescently labeled polynucleotide strand (SEQ ID NO.: 6) of the dsDNA HBV sequence complex prepared as described in section A of this Example. Fluorescence in relative fluorescence units (rfu) is plotted as a function of time (in seconds). The detection times, fluorescence emission peak intensities, emission colors (Cy3b-dCTP = 571 nm = Red), and Cy5-dUTP = 670 nm = Green) are summarized in the following Table 5, in which C is cytidine and U is uridine.

**Table 5**

| **Peak Time (sec)** | **Peak Intensity (rfu)** | **Emitted Color** | **Nucleotide Called** | **Nucleotide Position (5' to 3')** |
|---|---|---|---|---|
| 2.4 | 28.0 | Green | C | 180 |
| 6.3 | 10.0 | Red | U | 179 |
| 9.4 | 12.7 | Red | U | 176 |
| 11.2 | 11.8 | Red | U | 173 |
| 11.7 | 15.1 | Green | C | 172 |
| 14.6 | 25.0 | Green | C | 171 |
| 17.4 | 17.0 | Red | U | 165 |
| 18.5 | 8.0 | Green | C | 164 |

As can be seen, the time order and fluorescence emission wavelengths of the fluorescent signals matched the order (from the 3'- to 5'-direction) of cytidyl and uridyl nucleotides in SEQ ID NO.: 6, starting with the first 3'-mononucleotide of that fluorescently labeled strand. The variations in the fluorescent signal intensities measured for the various mononucleotides that were cleaved by the 3'-exonuclease were dependent in part on the random photobleaching times of individual fluorophores. The variable time intervals between released fluorescently labeled mononucleotides reflect the stochastic nature of single molecule exonucleolytic digestion of DNA, and are consistent with a catalytic cleavage rate of about one mononucleotide released per one to three seconds under the conditions of this experiment.

## Claims

1. A method for determining a nucleotide sequence of a polynucleotide comprising: providing
(a) a solid state substrate comprising a cis side and a trans side, the substrate comprising a reaction well that defines a reaction volume and comprises
(i) a proximal throughhole extending between the cis side and the trans side of the substrate,
(ii) one or more side walls, and
(iii) a distal opening,
wherein the solid state substrate further comprises an opaque metal layer that substantially blocks excitation light from penetrating into the reaction volume and from penetrating to the cis side of the substrate, and
(b) a carrier particle comprising a fluorescently labeled polynucleotide strand that is attached to the carrier particle, wherein the fluorescently labeled polynucleotide strand comprises
(i) a proximal end that is attached to the carrier particle,
(ii) a distal end that is cleavable by an exonuclease, and
(iii) at least one fluorescently labeled nucleotide comprising a fluorescent label, wherein the carrier particle is located on the cis side of the substrate, but does not pass through the throughhole, such that the fluorescently labeled polynucleotide strand protrudes through the throughhole so that the distal end of the fluorescently labeled polynucleotide strand is in the reaction volume;
reacting the fluorescently labeled polynucleotide strand with an exonuclease so that mononucleotides are released serially from the distal end of the strand and diffuse out of the reaction volume through the distal opening;
during said reacting, illuminating the trans side of the substrate with excitation light to create a fluorescence excitation zone adjacent to the distal opening of the reaction well, so that fluorescently labeled mononucleotides in the excitation zone emit fluorescent signals;
and detecting the fluorescent signals as a function of time;
whereby a nucleotide sequence is determined from the time order of fluorescent signals detected from the released fluorescently labeled mononucleotides.

2. The method of claim 1, wherein the distal opening of the reaction well has a minimum diameter of at least 30 nm, optionally
wherein the distal opening of the reaction well has a minimum diameter of 50 to 150 nm.

3. The method of claim 1 or claim 2, wherein the one or more walls of the reaction well are a) not tapered and/or b) substantially cylindrical.

4. The method of any one of the preceding claims, wherein the opaque metal layer a) comprises gold or aluminium and/or b) has a thickness of 100 nm to 600 nm.

5. The method of any one of the preceding claims, wherein the reaction well has a well depth of at least 200 nm, optionally wherein the reaction well has a well depth of 200 nm to 1000 nm.

6. The method of any one of the preceding claims, wherein the fluorescently labeled polynucleotide strand in the reaction volume comprises:
a) a fluorescently labeled polynucleotide segment containing at least 100 contiguous nucleotides; and/or
b) a double-stranded nucleic acid or a single-stranded nucleic acid.

7. The method of any one of the preceding claims, wherein the throughhole has a minimum diameter of at least 2 nm, optionally wherein the throughhole has a minimum diameter of 2 nm to 50 nm.

8. The method of any one of the preceding claims, wherein the substrate comprises a thin membrane layer that contains the proximal throughhole and has a thickness of between 20 nm and 50 nm, optionally wherein the thin membrane layer comprises silicon nitride.

9. The method of any one of the preceding claims, wherein the excitation light has a wavelength of 380 nm or greater.

10. The method of any one of the preceding claims, wherein the solid substrate comprises surface portion(s) that define the reaction volume, and the surface portion(s) comprise at least one surface passivation coating.

11. The method of any one of the preceding claims, wherein one or more side walls of the reaction well comprises at least one of a silicon oxide coating and an aluminum oxide coating.

12. The method of any one of the preceding claims, wherein the fluorescently labeled polynucleotide strand comprises at least two different kinds of nucleotides, each kind labeled with a distinguishing fluorescent label.

13. The method of any one of the preceding claims, wherein during said reacting, the carrier particle is maintained next to the proximal throughhole by a voltage bias, optionally wherein after said reacting, the voltage bias is stopped to allow the carrier particle to move away from the proximal throughhole, so that the remaining fluorescently labeled polynucleotide strand is removed from the reaction volume, and then a voltage bias is applied to move the same or a different carrier particle toward the proximal throughhole so that a new fluorescently labeled polynucleotide strand is delivered into the reaction well for reacting with an exonuclease.

14. The method of any one of the preceding claims, wherein the carrier particle:
a) is magnetic or not magnetic; and/or
b) comprises a plurality of fluorescently labeled polynucleotide strands, optionally having polynucleotide sequences that are different from each other.

15. The method of any one of the preceding claims, wherein the solid state substrate comprises a plurality of reaction wells, optionally wherein the plurality of reaction wells are configured as a one- dimensional or two-dimensional array and/or wherein two or more of the plurality of reaction wells each contain a fluorescently labeled polynucleotide strand to be sequenced.

## Patentansprüche

1. Ein Verfahren zum Bestimmen einer Nukleotidsequenz eines Polynukleotids, das Folgendes beinhaltet:
Bereitstellen
(a) eines Festkörpersubstrats, das eine cis-Seite und eine trans-Seite beinhaltet, wobei das Substrat eine Reaktionsmulde beinhaltet, die ein Reaktionsvolumen definiert und Folgendes beinhaltet:
(i) ein proximales Durchgangsloch, das sich zwischen der cis-Seite und der trans-Seite des Substrats erstreckt,
(ii) eine oder mehrere Seitenwände, und
(iii) eine distale Öffnung,
wobei das Festkörpersubstrat ferner eine opake Metallschicht beinhaltet, die im Wesentlichen verhindert, dass Anregungslicht in das Reaktionsvolumen eindringt und zu der cis-Seite des Substrats eindringt, und
(b) ein Trägerpartikel, das einen fluoreszierend markierten Polynukleotidstrang beinhaltet, der an das Trägerpartikel gebunden ist, wobei der fluoreszierend markierte Polynukleotidstrang Folgendes beinhaltet:
(i) ein proximales Ende, das an das Trägerpartikel gebunden ist,
(ii) ein distales Ende, das durch eine Exonuklease spaltbar ist, und
(iii) mindestens ein fluoreszierend markiertes Nukleotid, das eine fluoreszierende Markierung beinhaltet, wobei sich das Trägerpartikel auf der cis-Seite des Substrats befindet, aber nicht durch das Durchgangsloch verläuft, sodass der fluoreszierend markierte Polynukleotidstrang durch das Durchgangsloch vorragt, sodass sich das distale Ende des fluoreszierend markierten Polynukleotidstrangs in dem Reaktionsvolumen befindet;
Zur-Reaktion-Bringen des fluoreszierend markierten Polynukleotidstrangs mit einer Exonuklease, sodass Mononukleotide seriell von dem distalen Ende des Strangs freigesetzt werden und durch die distale Öffnung aus dem Reaktionsvolumen diffundieren;
während des Zur-Reaktion-Bringens, Beleuchten der trans-Seite des Substrats mit Anregungslicht, um eine Fluoreszenzanregungszone angrenzend an die distale Öffnung der Reaktionsmulde zu erzeugen, sodass fluoreszierend markierte Mononukleotide in der Anregungszone fluoreszierende Signale emittieren;
und Detektieren der fluoreszierenden Signale als eine Funktion der Zeit;
wodurch eine Nukleotidsequenz aus der zeitlichen Reihenfolge von fluoreszierenden Signalen bestimmt wird, die von den freigesetzten fluoreszierend markierten Mononukleotiden detektiert werden.

2. Verfahren gemäß Anspruch 1, wobei die distale Öffnung der Reaktionsmulde einen minimalen Durchmesser von mindestens 30 nm aufweist, wobei optional die distale Öffnung der Reaktionsmulde einen minimalen Durchmesser von 50 bis 150 nm aufweist.

3. Verfahren gemäß Anspruch 1 oder Anspruch 2, wobei die eine oder die mehreren Wände der Reaktionsmulde a) nicht verjüngt sind und/oder b) im Wesentlichen zylindrisch sind.

4. Verfahren gemäß einem der vorhergehenden Ansprüche, wobei die opake Metallschicht a) Gold oder Aluminium beinhaltet und/oder b) eine Dicke von 100 nm bis 600 nm aufweist.

5. Verfahren gemäß einem der vorhergehenden Ansprüche, wobei die Reaktionsmulde eine Muldentiefe von mindestens 200 nm aufweist, wobei optional die Reaktionsmulde eine Muldentiefe von 200 nm bis 1000 nm aufweist.

6. Verfahren gemäß einem der vorhergehenden Ansprüche, wobei der fluoreszierend markierte Polynukleotidstrang in dem Reaktionsvolumen Folgendes beinhaltet:
a) ein fluoreszierend markiertes Polynukleotidsegment, das mindestens 100 zusammenhängende Nukleotide enthält; und/oder
b) eine doppelsträngige Nukleinsäure oder eine einzelsträngige Nukleinsäure.

7. Verfahren gemäß einem der vorhergehenden Ansprüche, wobei das Durchgangsloch einen minimalen Durchmesser von mindestens 2 nm aufweist, wobei optional das Durchgangsloch einen minimalen Durchmesser von 2 nm bis 50 nm aufweist.

8. Verfahren gemäß einem der vorhergehenden Ansprüche, wobei das Substrat eine dünne Membranschicht beinhaltet, die das proximale Durchgangsloch enthält und eine Dicke von zwischen 20 nm und 50 nm aufweist, wobei optional die dünne Membranschicht Siliciumnitrid beinhaltet.

9. Verfahren gemäß einem der vorhergehenden Ansprüche, wobei das Anregungslicht eine Wellenlänge von 380 nm oder mehr aufweist.

10. Verfahren gemäß einem der vorhergehenden Ansprüche, wobei das Festkörpersubstrat einen oder mehrere Oberflächenabschnitte beinhaltet, die das Reaktionsvolumen definieren, und der eine oder die mehreren Oberflächenabschnitte mindestens eine Oberflächenpassivierungsbeschichtung beinhalten.

11. Verfahren gemäß einem der vorhergehenden Ansprüche, wobei eine oder mehrere Seitenwände der Reaktionsmulde mindestens eine von einer Siliciumoxidbeschichtung und einer Aluminiumoxidbeschichtung beinhalten.

12. Verfahren gemäß einem der vorhergehenden Ansprüche, wobei der fluoreszierend markierte Polynukleotidstrang mindestens zwei verschiedene Arten von Nukleotiden beinhaltet, wobei jede Art mit einer unterscheidenden fluoreszierenden Markierung markiert ist.

13. Verfahren gemäß einem der vorhergehenden Ansprüche, wobei während des Zur-Reaktion-Bringens das Trägerpartikel durch eine Vorspannung neben dem proximalen Durchgangsloch gehalten wird, wobei optional nach dem Zur-Reaktion-Bringen die Vorspannung gestoppt wird, um es dem Trägerpartikel zu ermöglichen, sich von dem proximalen Durchgangsloch wegzubewegen, sodass der verbleibende fluoreszierend markierte Polynukleotidstrang aus dem Reaktionsvolumen entfernt wird, und dann eine Vorspannung angelegt wird, um dasselbe oder ein anderes Trägerpartikel in Richtung des proximalen Durchgangslochs zu bewegen, sodass ein neuer fluoreszierend markierter Polynukleotidstrang in die Reaktionsmulde zur Reaktion mit einer Exonuklease abgegeben wird.

14. Verfahren gemäß einem der vorhergehenden Ansprüche, wobei das Trägerpartikel:
a) magnetisch oder nicht magnetisch ist; und/oder
b) eine Vielzahl von fluoreszierend markierten Polynukleotidsträngen beinhaltet, die optional Polynukleotidsequenzen aufweisen, die sich voneinander unterscheiden.

15. Verfahren gemäß einem der vorhergehenden Ansprüche, wobei das Festkörpersubstrat eine Vielzahl von Reaktionsmulden beinhaltet, wobei optional die Vielzahl von Reaktionsmulden als ein eindimensionales oder zweidimensionales Array konfiguriert ist und/oder wobei zwei oder mehr der Vielzahl von Reaktionsmulden jeweils einen fluoreszierend markierten Polynukleotidstrang enthalten, der zu sequenzieren ist.

## Revendications

1. Un procédé pour déterminer une séquence nucléotidique d'un polynucléotide comprenant:
le fait de fournir
(a) un substrat à l'état solide comprenant un côté cis et un côté trans, le substrat comprenant un puits de réaction qui définit un volume de réaction et comprend
(i) un trou traversant proximal s'étendant entre le côté cis et le côté trans du substrat,
(ii) une ou plusieurs parois latérales, et
(iii) une ouverture distale,
dans lequel le substrat à l'état solide comprend en outre une couche métallique opaque qui bloque substantiellement la pénétration de lumière d'excitation à l'intérieur du volume de réaction et la pénétration jusqu'au côté cis du substrat, et
(b) une particule de support comprenant un brin polynucléotidique marqué par fluorescence qui est fixé à la particule de support, dans lequel le brin polynucléotidique marqué par fluorescence comprend
(i) une extrémité proximale qui est fixée à la particule de support,
(ii) une extrémité distale qui est clivable par une exonucléase, et
(iii) au moins un nucléotide marqué par fluorescence comprenant un marqueur fluorescent, dans lequel la particule de support est située sur le côté cis du substrat, mais ne passe pas à travers le trou traversant, de telle sorte que le brin polynucléotidique marqué par fluorescence fait saillie à travers le trou traversant de sorte que l'extrémité distale du brin polynucléotidique marqué par fluorescence se trouve dans le volume de réaction ;
le fait de mettre en réaction le brin polynucléotidique marqué par fluorescence avec une exonucléase de sorte que des mononucléotides sont libérés en série à partir de l'extrémité distale du brin et se diffusent en dehors du volume de réaction à travers l'ouverture distale ;
durant ladite mise en réaction, le fait d'éclairer le côté trans du substrat avec de la lumière d'excitation afin de créer une zone d'excitation par fluorescence adjacente à l'ouverture distale du puits de réaction, de sorte que des mononucléotides marqués par fluorescence dans la zone d'excitation émettent des signaux fluorescents ;
et le fait de détecter les signaux fluorescents en fonction du temps ;
moyennant quoi une séquence nucléotidique est déterminée à partir de l'ordre chronologique de signaux fluorescents détectés en provenance des mononucléotides marqués par fluorescence libérés.

2. Le procédé de la revendication 1, dans lequel l'ouverture distale du puits de réaction a un diamètre minimal d'au moins 30 nm, facultativement dans lequel l'ouverture distale du puits de réaction a un diamètre minimal allant de 50 à 150 nm.

3. Le procédé de la revendication 1 ou de la revendication 2, dans lequel les une ou plusieurs parois du puits de réaction sont a) non biseautées et/ou b) substantiellement cylindriques.

4. Le procédé de l'une quelconque des revendications précédentes, dans lequel la couche métallique opaque a) comprend de l'or ou de l'aluminium et/ou b) a une épaisseur allant de 100 nm à 600 nm.

5. Le procédé de l'une quelconque des revendications précédentes, dans lequel le puits de réaction a une profondeur de puits d'au moins 200 nm, facultativement dans lequel le puits de réaction a une profondeur de puits allant de 200 nm à 1 000 nm.

6. Le procédé de l'une quelconque des revendications précédentes, dans lequel le brin polynucléotidique marqué par fluorescence dans le volume de réaction comprend :
a) un segment polynucléotidique marqué par fluorescence contenant au moins 100 nucléotides contigus ; et/ou
b) un acide nucléique double brin ou un acide nucléique simple brin.

7. Le procédé de l'une quelconque des revendications précédentes, dans lequel le trou traversant a un diamètre minimal d'au moins 2 nm, facultativement dans lequel le trou traversant a un diamètre minimal allant de 2 nm à 50 nm.

8. Le procédé de l'une quelconque des revendications précédentes, dans lequel le substrat comprend une couche membranaire mince qui contient le trou traversant proximal et a une épaisseur comprise entre 20 nm et 50 nm, facultativement dans lequel la couche membranaire mince comprend du nitrure de silicium.

9. Le procédé de l'une quelconque des revendications précédentes, dans lequel la lumière d'excitation a une longueur d'onde de 380 nm ou plus.

10. Le procédé de l'une quelconque des revendications précédentes, dans lequel le substrat solide comprend une ou des portions de surface qui définissent le volume de réaction, et la ou les portions de surface comprennent au moins un revêtement de passivation de surface.

11. Le procédé de l'une quelconque des revendications précédentes, dans lequel une ou plusieurs parois latérales du puits de réaction comprennent au moins soit un revêtement d'oxyde de silicium, soit un revêtement d'oxyde d'aluminium.

12. Le procédé de l'une quelconque des revendications précédentes, dans lequel le brin polynucléotidique marqué par fluorescence comprend au moins deux types différents de nucléotides, chaque type étant marqué avec un marqueur fluorescent distinctif.

13. Le procédé de l'une quelconque des revendications précédentes, dans lequel, durant ladite mise en réaction, la particule de support est maintenue à proximité du trou traversant proximal par une polarisation en tension, facultativement dans lequel, après ladite mise en réaction, la polarisation en tension est arrêtée afin de permettre à la particule de support de s'écarter du trou traversant proximal, de sorte que le brin polynucléotidique marqué par fluorescence restant est retiré du volume de réaction, et ensuite une polarisation en tension est appliquée afin de déplacer la même particule de support ou une particule de support différente vers le trou traversant proximal de sorte qu'un nouveau brin polynucléotidique marqué par fluorescence est distribué à l'intérieur du puits de réaction pour réagir avec une exonucléase.

14. Le procédé de l'une quelconque des revendications précédentes, dans lequel la particule de support :
a) est magnétique ou non magnétique ; et/ou
b) comprend une pluralité de brins polynucléotidiques marqués par fluorescence, ayant facultativement des séquences polynucléotidiques qui sont différentes les unes des autres.

15. Le procédé de l'une quelconque des revendications précédentes, dans lequel le substrat à l'état solide comprend une pluralité de puits de réaction, facultativement dans lequel la pluralité de puits de réaction sont configurés sous la forme d'un réseau unidimensionnel ou bidimensionnel et/ou dans lequel deux puits de réaction ou plus de la pluralité de puits de réaction contiennent chacun un brin polynucléotidique marqué par fluorescence destiné à être séquencé.
